# EUROPEAN PATENT SPECIFICATION

(11) **EP 4 522 171 B1**
(45) Date of publication and mention of the grant of the patent: **29.07.2026**
(21) Application number: 23726357.9
(22) Date of filing: 11.05.2023
(51) Int. Cl.: A61K 31/4725, A61K 31/519, A61P 35/00, G01N 33/50

(54) **COMBINATION THERAPY FOR CANCER COMPRISING A CDC7 INHIBITOR AND A CDK8 INIHIBITOR OR A CCNC INHIBITOR**
KOMBINATIONSTHERAPIE GEGEN KREBS MIT EINEM CDC7-INHIBITOR UND EINEM CDK8-INHIBITOR ODER EINEM CCNC-INHIBITOR
THÉRAPIE COMBINÉE POUR LE CANCER COMPRENANT UN INHIBITEUR DE CDC7 ET UN INHIBITEUR DE CDK8 OU UN INHIBITEUR DE CCNC

(30) Priority: 11.05.2022 EP 22172868
(43) Date of publication of application: 19.03.2025
(73) Proprietor: National University of Ireland Galway, Galway (IE)
(72) Inventor: SANTOCANALE, Corrado, Galway University Road Galway (IE); RAINEY, Michael David, Galway University Road Galway (IE)
(74) Representative: Barker Brettell LLP
(86) International application number: PCT/EP2023/062643
(87) International publication number: WO 2023/217980

(56) References cited:
- WO-A1-2019/165473
- HOFMANN MARCO H. ET AL: "Selective and Potent CDK8/19 Inhibitors Enhance NK-Cell Activity and Promote Tumor Surveillance", vol. 19, no. 4, 1 April 2020 (2020-04-01), US, pages 1018 - 1030, XP055976400, ISSN: 1535-7163, Retrieved from the Internet <URL:https://aacrjournals.org/mct/article-pdf/19/4/1018/1849306/1018.pdf> DOI: 10.1158/1535-7163.MCT-19-0789

## Description

The present invention relates to a combination therapy to treat or prevent cancer, including methods of treatment or prevention, uses, compositions and kits for treating cancer in a subject.

### Background of the Invention

During tumorigenesis, oncogene activation results in dysregulation of DNA replication and transcription programmes ultimately driving the hyper-proliferative state of cancer cells. Cell Division Cycle 7 (CDC7) kinase encoded by the *CDC7* gene is considered to be a key switch for DNA replication and its depletion by siRNA causes cancer cells to stop DNA synthesis. As such, a number of CDC7 kinase inhibitors (termed "CDC7i" herein) are in development as a novel class of anti-cancer treatment. Such clinical studies include (ClinicalTrials.gov):
1. Study of BMS-863233 in patients with hematologic cancer: Terminated
2. Study of BMS-863233 in patients with advanced and/or metastatic solid tumors: Terminated
3. CR-UK phase I trial of LY3143921: Recruiting
4. Clinical study of TQB3824 in subjects with advanced cancer: Recruiting
5. Study of NMS-1116354 in solid tumors: Terminated
6. Study of NMS-1116354 in advanced/metastatic solid tumors: Terminated
7. Study to evaluate TAK-931 in participants with advanced nonhematologic tumors: completed

However, problems persist in this approach where there are ongoing issues with reaching a good level of efficacy whilst avoiding toxicity of these CDC7 kinase inhibitors. In particular, tolerability issues have been observed with some inhibitors leading to their clinical trial termination.

Most likely CDC7 inhibitors will be included as a component of chemotherapy with other agents. WO 2019/165473 A1 (2019-08-29) discloses that treatment with the CDC7 inhibitor SRA141 sensitizes cancer cells to treatment with the MAPK pathway inhibitor trametinib, with the PI3K pathway inhibitor copanlisib, with the PARP inhibitor BMN673, with the Bcl-2 inhibitor ABT-199, with the mTOR inhibitor everolimus or with the RXR agonist bexarotene. Previous studies have shown that inhibition of the ATR kinase can modify the cell sensitivity to CDC7i, however enhancement of CDC7i anti-cancer activity is observed only at very high doses of CDC7i while suppression is observed at lower doses (Guo et al., 2021; Iwai et al., 2019; Rainey et al., 2020). Such a combination therapy does not adequately address the clinical need.

Thus, there is a clear clinical need for more novel therapeutic agents for cancer treatment and an ongoing need to find alternative and improved therapies relative to those under study.

An aim of the present description is to provide an alternative or improved therapy for cancer.

### Summary of the Invention

Any reference to methods of treatment or prevention applied on the human or animal body for therapeutic purposes as made in the description of the claimed invention is to be construed as reference to the compounds, pharmaceutical compositions and medicaments of the present invention for use in such a method of treatment or prevention.

According to a first aspect of the present invention, there is provided a Cell Division Cycle 7 kinase inhibitor for the use according to claim 1 herein.

Also described is a method of treatment or prevention of cancer in a subject comprising the administration of a Cell Division Cycle 7 kinase inhibitor (CDC7i) in combination with:
i) a Cyclin Dependent Kinase 8 inhibitor (CDK8i), and/or
ii) a Cyclin C inhibitor (CCNCi).

The description advantageously identifies CDK8 as a protein, that assists CDC7 in DNA replication. It is found that pharmacological inhibition of CDK8 has a negligible effect on DNA replication and cell division, but greatly enforces the anti-proliferative activity of CDC7 inhibitors when used in combination. Thus, the use of CDK8 inhibitors (and/or inhibitors of its cognate CCNC) in combination with CDC7 inhibitors greatly improve the efficacy of the latter anti-cancer drug. CDK8 inhibitors are being developed as potential anti-cancer agents with 2 reported clinical trials (ClinicalTrials.gov):
1. Study of BCD-115 in women with ER(+) HER2(-) local advanced and metastatic breast cancer: completed (treatment discontinuation due to adverse events)
2. RVU120(SEL120) in patients with acute myeloid leukaemia or high-risk myelodysplastic syndrome: Phase I; recruiting.

However, despite the typical monotherapeutic approach for treatment of cancer, the present description finds for the first time that CDC7i and CDK8i (additionally or alternative CCNCi) synergise in stopping DNA replication and cell proliferation. Therefore, combination therapy using CDC7i and CDK8i can be more efficacious and less toxic than CDC7 inhibitor monotherapy for the treatment of solid cancers, blood cancers and other proliferative diseases.

### The Cell Division Cycle 7 kinase inhibitor (CDC7i)

The CDC7i may bind to CDC7. In one embodiment, the binding to CDC7 is preferential or specific binding.

The CDC7i may be a small molecule (i.e. less than 900Da). The CDC7i may be a small molecule ATP-competitive CDC7 inhibitor,

The CDC7i may comprise or consist of XL413, or a functional derivative or variant thereof having CDC7 inhibition activity.

The CDC7i may comprise or consist of any one of the group of CDC7 inhibitors selected from PHA-767491 (Montagnoli et al., 2008), XL413 (BMS-863233; Clinicaltrials.gov ID: NCT00838890 or NCT00886782) (Koltun et al., 2012), AS-0141 (Irie et al., 2021), Dequalinium Chloride, Clofoctol (Cheng et al., 2018), LY3143921 (Clinicaltrials.gov ID: NCT03096054), TQB3824 (Clinicaltrials.gov ID: NCT05028218), NMS-1116354 (Clinicaltrials.gov ID: NCT01016327 or NCT01092052) and TAK-931 (Clinicaltrials.gov ID: NCT02699749) (Iwai et al., 2019); or combinations thereof.

PHA-767491 (PubChem CID: 11715767) has the formula:

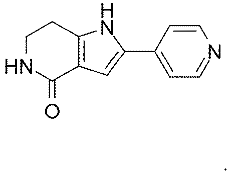

Active variants of PHA-767491 can be found in Vanotti et al. (Vanotti et al., 2008), Menichincheri et al. (Menichincheri et al., 2009), Ermoli et al. (Ermoli et al., 2009), and Menichincheri et al. (Menichincheri et al., 2010).

XL413 (BMS-863233, PubChem CID: 135564632) has the formula:

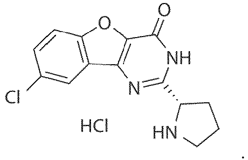

Active variants of XL413 can be found in Koltun et al. (Koltun et al., 2012).

AS-0141 (PubChem CID: 136618371) has the formula:

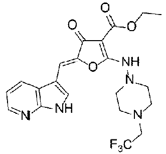

Active variants of AS-0141 can be found in Irie et al. (Irie et al., 2017); and Irie et al. (Irie et al., 2021).

Dequalinium chloride (DQC, PubChem CID: 2993) has the formula:

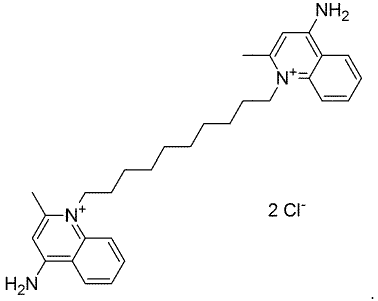

Clofoctol (PubChem CID: 2799) has the formula:

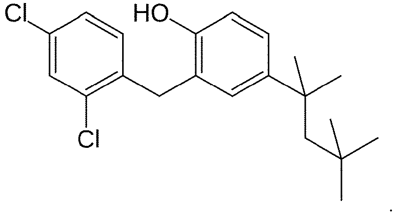

LY3143921 (PubChem CID: 137794949) has the formula:

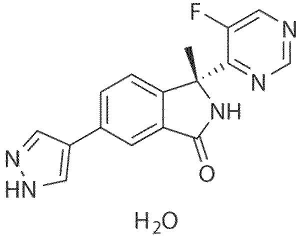

TQB3203 has the formula:

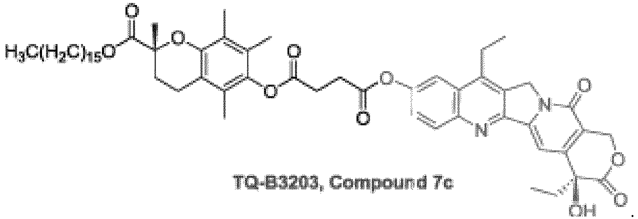

NMS-1116354 has the formula:

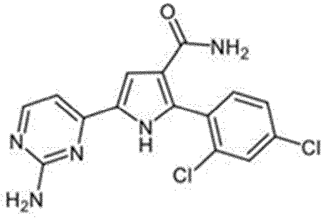

TAK-931 (Simurosurtib, PubChem CID: 136603526) has the formula:

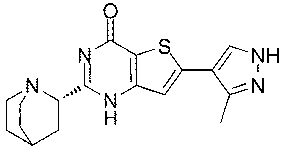

TAK-931 is described in Iwai K et al. (Iwai et al., 2019). Active variants of TAK-931 may be found in Kurasawa et al. (Kurasawa et al., 2020) and Kurasawa et al. (Kurasawa et al., 2017).

In a preferred embodiment, the CDC7i comprises or consists of TAK931 or AS-0114. In one embodiment, the CDC7i comprises or consists of TAK931. In another embodiment, the CDC7i comprises or consists of AS-0114. In another preferred embodiment, the CDC7i comprises or consists ofXL413 (BMS-863233).

The skilled person will recognise that functional derivatives or variants of these CDC7i molecules may be available for use.

In another embodiment, the CDC7i is a nucleic acid, such as an inhibitory RNA molecule. The CDC7i may be an siRNA capable of inhibiting the expression of CDC7, such as inhibiting the transcription of the gene encoding CDC7 or inhibiting translation of mRNA encoding CDC7. The CDC7i, such as a siRNA, may bind the sequence encoding CDC7 or a complementary sequence thereof. The siRNA may be 20-30 nucleotides long. The siRNA may comprise or consist of RNA or may comprise or consist of nucleotide analogues. The CDC7i may specifically bind (i.e. hybridise with) a sequence comprising or consisting of SEQ ID NO: 1. The CDC7i may be any CDC7 siRNA described herein.

### The Cyclin Dependent Kinase 8 inhibitor (CDK8i)

The CDK8i may be a dual CDK8/CDK19 inhibitor. In another embodiment, the CDK8i inhibitor may be specific for CDK8 and not CDK19. The CDK8i may not significantly inhibit CDK19.

The CDK8i may comprise or consist of BI1347, or a functional derivative or variant thereof having CDK8 inhibition activity.

The CDK8i may comprise or consist of any one of the group of CDK8 inhibitors selected from BI1347 (Hofmann et al., 2020), RVU120 (SEL120-34A, Clinicaltrials.gov ID: NCT04021368) (Johannessen et al., 2017), Senexin A (Porter et al., 2012), BCD-115 (Senexin B, Clinicaltrials.gov ID: NCT03065010) (Johannessen et al., 2017), Senexin C (Zhang et al., 2022), MSC2530818 (Czodrowski et al., 2016; Mallinger et al., 2016), AS2863619 (Akamatsu et al., 2019), 16-didehydro-cortistatin A (DCA) (Arnett et al., 2021), BRD6989 (Johannessen et al., 2017), CCT-251545 (Mallinger et al., 2015), CCT-251921 (Mallinger et al., 2016), TSN084 (Clinicaltrials.gov ID: NCT05300438), JH-XVI-178 (Hatcher et al., 2021), and JH-XI-10-02 (Hatcher et al., 2018); or combinations thereof.

Active variants of the CDK8i compounds may be found in Mallinger et al. (Mallinger et al., 2015) (e.g. for CCT251545); Mallinger et al. (Mallinger et al., 2016) (e.g. for CCT251921 or Compound 42 / MSC2530818); Czodrowski et al., (Czodrowski et al., 2016)(e.g. for MSC2530818); Clarke et al. (Clarke et al., 2016)_(e.g. for CCT251921 or MSC2530818). CDK8 inhibitors are also described in Ettl et al. (Ettl et al., 2022).

BI1347 (PubChem CID: 132180482) has the formula:

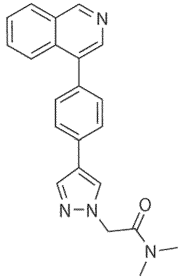

RVU120 (SEL120-34A, PubChem CID: 73776233) has the formula:

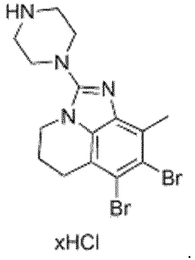

Senexin A (PubChem CID: 56927063) has the formula:

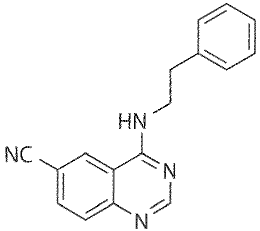

BCD-115 (Senexin B, PubChem CID: 71661259) has the formula:

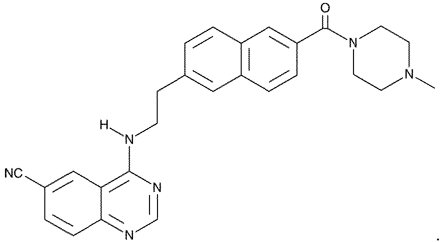

Senexin C has the formula:

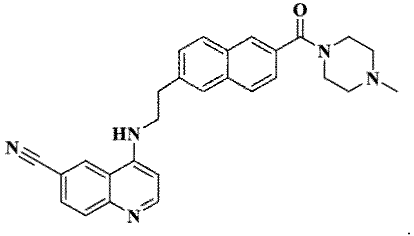

Active variants of Senexin C can be found in Zhang et al. (Zhang et al., 2022). MSC2530818 (PubChem CID: 118879529) has the formula:

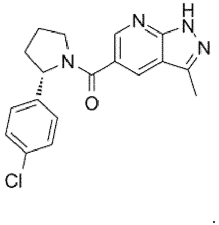

AS2863619 (PubChem CID: 139600293) has the formula:

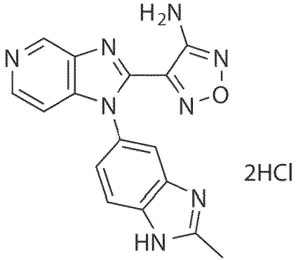

16-didehydro-cortistatin A has the formula:

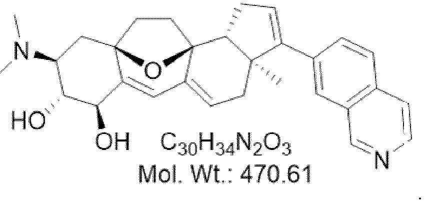

BRD6989 (PubChem CID: 3835861) has the formula:

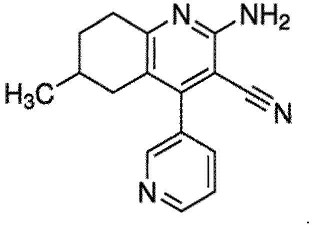

CCT-251545 (PubChem CID: 77050682) has the formula:

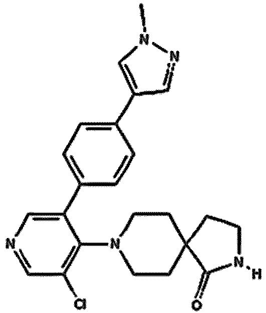

CCT-251921 (PubChem CID: 74222277) has the formula:

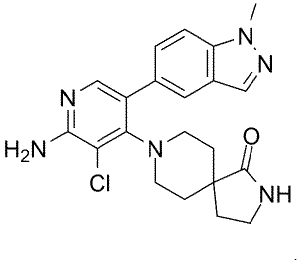

JH-XVI-178 has the formula:

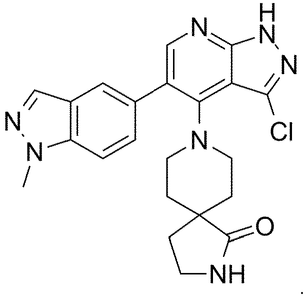

JH-XI-10-02 (PubChem CID: 133081965) has the formula:

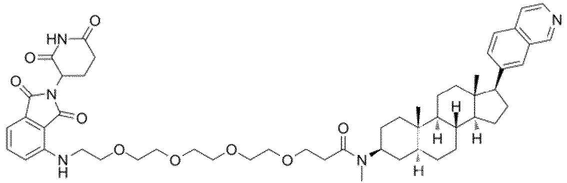

The skilled person will recognise that functional derivatives or variants of these CDK8i molecules may be available for use.

In another embodiment, the CDK8i is a nucleic acid, such as an inhibitory RNA molecule. The CDK8i may be an siRNA capable of inhibiting the expression of CDK8, such as inhibiting the transcription of the gene encoding CDK8 or inhibiting translation of mRNA encoding CDK8. The CDK8i, such as a siRNA, may bind the sequence encoding CDK8 or a complementary sequence thereof. The siRNA may be 20-30 nucleotides long. The siRNA may comprise or consist of RNA or may comprise or consist of nucleotide analogues. The CDK8i may specifically bind (i.e. hybridise with) a sequence comprising or consisting of SEQ ID NO: 2. The CDK8i may be any CDK8 siRNA described herein.

### The CCNC inhibitor (CCNCi)

In one embodiment, the CCNCi is a nucleic acid, such as an inhibitory RNA molecule. The CCNCi may be an siRNA capable of inhibiting the expression of CCNC, such as inhibiting the transcription of the gene encoding CCNCi or inhibiting translation of mRNA encoding CCNCi. The CCNCi, such as a siRNA, may bind the sequence encoding CCNC or a complementary sequence thereof. The siRNA may be 20-30 nucleotides long. The siRNA may comprise or consist of RNA or may comprise or consist of nucleotide analogues. The CCNCi may specifically bind (i.e. hybridise with) a sequence comprising or consisting of SEQ ID NO: 3 The CCNCi may be any CCNC siRNA described herein.

### Derivatives of CDC7i and/or CDK8i

It will generally be appreciated that the above inhibitors are specific examples of compounds that are known to inhibit CDC7 and/or CDK8. Derivatives and variants of these specific compounds are likely to work in a similar manner to these compounds, even if their activity is not optimal.

Reference to a particular compound also includes variants where in one or more tertiary amine of the CDC7i and/or CDK8i (i.e. amines that are bonded to no hydrogen atoms) the N atom is replaced by P. Similarly, a reference to a particular CDC7i and/or CDK8i compound also includes variants where in one or more secondary and/or primary amine(s) of the compound (i.e. amines that are bonded to one or two hydrogen atoms) the N atom is replaced by O, S or P. Reference to a particular compound also includes variants where primary amines and phosphines are functionalised by one or two R^{Z} groups, and/or where secondary amines and phosphines are functionalised by one R^{Z} group.

Reference to a particular compound also includes variants where alcohols and/or ethers present in the compounds are replaced by NH, NR^{Z}, S, PH or PR^{Z}.

Reference to a particular compound also includes variants where halogens (e.g. F, Cl, Br and I) are replaced with another halogen (e.g. F, Cl, Br or I), H, CN, NH₂, NHR^{Z} NR^{Z}₂, PH₂, PHR^{Z}, PR^{Z}₂, OH, OR^{Z} SH or SR^{Z}. Reference to a particular compound also includes variants where a nitrile group is replaced with a halogen (e.g. F, Cl, Br or I), H, OH, NH₂, NHR^{Z}, NR^{Z}₂, PH₂, PHR^{Z}, PR^{Z}₂, OR^{Z} or SR^{Z}.

Reference to a particular compound also includes variants where one or more primary amide is substituted by one or two are functionalised by one or two R^{Z} groups, and/or where secondary amides are functionalised by one R^{Z} group.

R^{Z} represents alkyl, alkenyl and/or aryl group(s). R^{Z} groups may be C1-8 alkyl, C2-8 alkenyl or C5-8 aryl group(s), for example C1-6 alkyl, C1-6 alkenyl or C5-6 aryl group(s), preferably C1-4 alkyl or C2-4 alkenyl group(s).

Reference to a particular compound also includes variants where one or more (e.g. two or more) of the hydrogen atoms in the hydrocarbon chain of an alkyl, alkenyl and/or alkynyl groups are replaced with substituent groups. In one embodiment from 1 to 10 hydrogen atoms in the group are substituted, such as from 1 to 6, e.g. 1, 2, 3 or 4 of the hydrogen atoms in the hydrocarbon chain might be replaced with substituent groups. When more than one hydrogen atom in the group is replaced, the substituent groups used may be the same or may be different. For example, the alkyl, alkenyl or alkynyl group may be substituted with one or more substituent groups independently selected from halo (e.g. F, Cl, Br and I), hydroxyl, amino and carboxyl groups, and aryl or heteroaryl groups (especially unsaturated cyclic and heterocyclic groups with 5 to 10 atoms (e.g. 6 to 10 atoms) in their ring, such as imidazolyl, thiazolyl, thienyl, phenyl, tolyl, xylyl, pyridinyl, pyrimidinyl, pyrazinyl, indolyl or naphthyl groups). The alkyl, alkenyl or alkynyl group may be substituted with one or more substituent groups independently selected from halo (e.g. F, Cl and Br) hydroxyl and amino and carboxyl groups. The alkyl, alkenyl or alkynyl group may be substituted with one or more substituent groups independently selected from aryl or heteroaryl groups, especially unsaturated cyclic and heterocyclic groups with 5 to 10 atoms (e.g. 6 to 10 atoms) in their ring, such as imidazolyl, thiazolyl, thienyl, phenyl, tolyl, xylyl, pyridinyl, pyrimidinyl, pyrazinyl, indolyl or naphthyl groups. The ring itself may be substituted, e.g. with one or more C1-6 alkyl groups, such as one or two (or more) methyl or ethyl groups, as is the case in tolyl and xylyl. Preferably the total number of carbon atoms in each of the substituent groups is from 5 to 12.

Reference to a particular compound also includes variants where the length of chains, such as alkyl and polyether (e.g. polyethylene glycol) chains are shortened or lengthened, for example by ten or fewer carbon atoms or polyether units, such as 6 or fewer, or 4 or fewer, or two (e.g. one) or fewer carbon atoms or polyether units. For example, a methyl group shown in a particular compound may be an ethyl group in a variant of the compound.

Reference to a particular compound also includes variants that are ionic forms, salts, solvates, conformers, isomers, tautomers, N-oxides, esters, prodrugs, isotopes and protected forms thereof, for example, as discussed below; preferably, ionic forms, or salts or tautomers or isomers or N-oxides or solvates thereof; more preferably, ionic forms, or salts or tautomers or solvates or protected forms thereof; even more preferably the salts or tautomers or solvates thereof. Many compounds, especially those including acidic and/or basic moieties, can exist in the form of salts, for example acid addition salts or, in certain cases salts of organic and inorganic bases such as carboxylate, sulphonate and phosphate salts. All such salts are within the scope of this description, and reference to a compound includes the salt forms of the compound.

The salt forms of the compounds of the description are typically pharmaceutically acceptable salts, and examples of pharmaceutically acceptable salts are discussed in Berge *et al.* (Berge et al., 1977). However, salts that are not pharmaceutically acceptable may also be prepared as intermediate forms which may then be converted into pharmaceutically acceptable salts. Such non-pharmaceutically acceptable salts forms, which may be useful, for example, in the purification or separation of the compounds of the description, also form part of the description.

The salts of the present description can be synthesized from the parent compound that contains a basic or acidic moiety by conventional chemical methods such as methods described in (Stahl et al., 2011; Wermuth and Stahl, 2002). Generally, such salts can be prepared by reacting the free acid or base forms of these compounds with the appropriate base or acid in water or in an organic solvent, or in a mixture of the two; generally, nonaqueous media such as ether, ethyl acetate, ethanol, isopropanol, or acetonitrile are used. The compounds of the description may exist as mono- or di-salts depending upon the pKa of the acid from which the salt is formed. Acid addition salts may be formed with a wide variety of acids, both inorganic and organic. Examples of acid addition salts include salts formed with an acid selected from the group consisting of acetic, hydrochloric, hydriodic, phosphoric, nitric, sulphuric, citric, lactic, succinic, maleic, malic, isethionic, fumaric, benzenesulphonic, toluenesulphonic, methanesulphonic (mesylate), ethanesulphonic, naphthalenesulphonic, valeric, acetic, propanoic, butanoic, malonic, glucuronic and lactobionic acids. Another group of acid addition salts includes salts formed from acetic, adipic, ascorbic, aspartic, citric, DL-Lactic, fumaric, gluconic, glucuronic, hippuric, hydrochloric, glutamic, DL-malic, methanesulphonic, sebacic, stearic, succinic and tartaric acids. If the compound is anionic, or has a functional group which may be anionic (e.g., -COOH may be -COO⁻), then a salt may be formed with a suitable cation. Examples of suitable inorganic cations include, but are not limited to, alkali metal ions such as Na⁺ and K⁺, alkaline earth metal cations such as Ca²⁺ and Mg²⁺, and other cations such as Al³⁺. Examples of suitable organic cations include, but are not limited to, ammonium ion (i.e., NH₄⁺) and substituted ammonium ions (e.g., NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

Where the CDC7i and/or CDK8i compounds contain an amine function, these may form quaternary ammonium salts, for example by reaction with an alkylating agent according to methods well known to the skilled person. Reference to a CDC7i and/or CDK8i compound includes reference to such quaternary ammonium compounds. CDC7i and/or CDK8i compounds containing an amine function may also form N-oxides. A reference herein to a CDC7i and/or CDK8i compound) that contains an amine function also includes the N-oxide. Where a compound contains several amine functions, one or more than one nitrogen atom may be oxidised to form an N-oxide. Particular examples of N-oxides are the N-oxides of a tertiary amine or a nitrogen atom of a nitrogen-containing heterocycle. N-Oxides can be formed as described in Advanced Organic Chemistry (Teesdale-Spittle, 1993) and by L. W. Deady (Deady, 1977).

The compounds of the description may form solvates, for example with water (i.e., hydrates) or common organic solvents. As used herein, the term "solvate" means a physical association of the compounds of the present description with one or more solvent molecules. This physical association involves varying degrees of intermolecular, ionic and covalent bonding, including hydrogen bonding. In certain instances, the solvate will be capable of isolation, for example when one or more solvent molecules are incorporated in the crystal lattice of the crystalline solid. The term "solvate" is intended to encompass both solution-phase and isolatable solvates. Nonlimiting examples of suitable solvates include compounds of the description in combination with water, isopropanol, ethanol, methanol, DMSO, ethyl acetate, acetic acid and ethanolamine. The compounds of the description may exert their biological effects whilst they are in solution. Furthermore, the compounds of the present description may have one or more polymorph (crystalline) or amorphous forms and as such are intended to be included in the scope of the description.

CDC7i and/or CDK8i compounds may exist in a number of different isomeric (e.g. geometrically isomeric or stereoisomeric), and tautomeric forms and references to those compounds include all such forms. For the avoidance of doubt, where a compound can exist in one of several geometric isomeric or tautomeric forms and only one is specifically described or shown, all others are nevertheless embraced the present description. Other examples of tautomeric forms include, for example, keto-, enol-, and enolate-forms, as in, for example, the following tautomeric pairs: keto/enol, imine/enamine, amide/imino alcohol, amidine/enediamines, nitroso/oxime, thioketone/enethiol, and nitro/aci-nitro.

Where CDC7i and/or CDK8i compounds contain one or more chiral centres, and can exist in the form of two or more optical isomers, references to those compounds include all optical isomeric forms thereof (e.g. enantiomers, epimers and diastereoisomers), either as individual optical isomers, or mixtures (e.g. racemic mixtures) of two or more optical isomers, unless the context requires otherwise. Where CDC7i and/or CDK8i compounds exist as two or more optical isomeric forms, one isomer (e.g. enantiomer) in a pair of isomers may exhibit advantages over the other enantiomer, for example, in terms of biological activity. Thus, in certain circumstances, it may be desirable to use as a therapeutic agent only one of a pair of isomers (e.g. enantiomers), or only one of a plurality of diastereoisomers. Accordingly, the description provides compositions containing a CDC7i and/or CDK8i compound having one or more chiral centres, wherein at least 55% (e.g. at least 60%, 65%, 70%, 75%, 80%, 85%, 90% or 95%) of the compound is present as a single optical isomer (e.g. enantiomer or diastereoisomer). In one embodiment, 99% or more (e.g. substantially all) of the total amount of the compound may be present as a single optical isomer (e.g. enantiomer or diastereoisomer).

The compounds of the description include compounds with one or more isotopic substitutions, and a reference to a particular element includes within its scope all isotopes of the element. For example, a reference to hydrogen includes within its scope ¹H, ²H (D), and ³H (T). Similarly, references to carbon and oxygen include within their scope respectively ¹²C, ¹³C and ¹⁴C and ¹⁶O and ¹⁸O.

Reference to CDC7i and/or CDK8i compounds may include reference to prodrugs thereof, for example esters such as carboxylic acid esters and acyloxy esters of the CDC7i and/or CDK8i compounds bearing a carboxylic acid group or a hydroxyl group. Esters are compounds containing the group -C(=O)OR, wherein R is an ester substituent, for example, a C₁₋₆ alkyl group, a heterocyclyl group, or a C₅₋₂₀ aryl group, preferably a C₁₋₆ alkyl group. Particular examples of ester groups include methyl, ethyl, t-butyl and phenyl esters. Examples of acyloxy (reverse ester) groups are represented by -OC(=O)R, wherein R is an acyloxy substituent, for example, a C₁₋₇ alkyl group, a C₃₋₂₀ heterocyclyl group, or a C₅₋₂₀ aryl group, preferably a C₁₋₇ alkyl group. Particular examples of acyloxy groups include -OC(=O)CH₃ (acetoxy), -OC(=O)CH₂CH₃, -OC(=O)C(CH₃)₃, -OC(=O)Ph, and -OC(=O)CH₂Ph. The term "prodrug" means any compound that is converted into a biologically active compound *in vivo.* For example, some prodrugs are esters of the active compound (e.g., a physiologically acceptable metabolically labile ester). The prodrug may be a sugar derivative or other glycoside conjugate, or may be an amino acid ester derivative. During metabolism, the ester group (-C(=O)OR), sugar derivative or other glycoside conjugate can be cleaved to yield the active drug.

### The subject

The subject may be mammalian, such as human. In one embodiment, the subject is a human patient afflicted with, or at risk of, cancer.

### Administration

The administration of the CDC7 inhibitor and CDK8 (and/or CCNC) inhibitor may be sequential, for example, immediately or within minutes, hours or days. Alternatively, the administration of the CDC7 inhibitor and CDK8 (and/or CCNC) inhibitor may be concurrent. The administration may be concurrent where the inhibitors are co-formulated.

In one embodiment, the CDC7 inhibitor and CDK8 (and/or CCNC) inhibitor are administered within one day of each other. In another embodiment, the CDC7 inhibitor and CDK8 (and/or CCNC) inhibitor may be administered within 48 hours of each other. In another embodiment, the CDC7 inhibitor and CDK8 (and/or CCNC) inhibitor may be administered within 35 hours of each other. The CDC7 inhibitor and CDK8 (and/or CCNC) inhibitor may be administered within 30 hours of each other. In another embodiment, the CDC7 inhibitor and CDK8 (and/or CCNC) inhibitor may be administered within 24 hours of each other. The CDC7 inhibitor and CDK8 (and/or CCNC) inhibitor may be administered within 16 hours of each other. In another embodiment, the CDC7 inhibitor and CDK8 (and/or CCNC) inhibitor may be administered within 12 hours of each other. The CDC7 inhibitor and CDK8 (and/or CCNC) inhibitor may be administered within 8 hours of each other. In another embodiment, the CDC7 inhibitor and CDK8 (and/or CCNC) inhibitor may be administered within 6 hours of each other. Preferably the CDC7 inhibitor and CDK8 inhibitor are administered within 1 hour of each other.

Where separate administration occurs, the CDC7 inhibitor and CDK8 (and/or CCNC) inhibitor may be administered at least 1 hour apart. In another embodiment, the CDC7 inhibitor and CDK8 (and/or CCNC) inhibitor may be administered at least 4 hours apart. In another embodiment, the CDC7 inhibitor and CDK8 (and/or CCNC) inhibitor may be administered at least 8 hours apart. In another embodiment, the CDC7 inhibitor and CDK8 (and/or CCNC) inhibitor may be administered at least 12 hours apart. In another embodiment, the CDC7 inhibitor and CDK8 (and/or CCNC) inhibitor may be administered at least 18 hours apart. In another embodiment, the CDC7 inhibitor and CDK8 (and/or CCNC) inhibitor may be administered at least 24 hours apart.

The CDC7 inhibitor and CDK8 inhibitor may be administered between about 4 hours and about 48 hours apart. The CDC7 inhibitor and CDK8 (and/or CCNC) inhibitor may be administered between about 8 hours and about 48 hours apart. The CDC7 inhibitor and CDK8 (and/or CCNC) inhibitor may be administered between about 12 hours and about 48 hours apart. The CDC7 inhibitor and CDK8 (and/or CCNC) inhibitor may be administered between about 4 hours and about 30 hours apart. The CDC7 inhibitor and CDK8 (and/or CCNC) inhibitor may be administered between about 4 hours and about 24 hours apart. The CDC7 inhibitor and CDK8 (and/or CCNC) inhibitor may be administered between about 8 hours and about 30 hours apart. The CDC7 inhibitor and CDK8 (and/or CCNC) inhibitor may be administered between about 8 hours and about 24 hours apart. The B CDC7 inhibitor and CDK8 (and/or CCNC) inhibitor may be administered between about 12 hours and about 30 hours apart. The CDC7 inhibitor and CDK8 (and/or CCNC) inhibitor may be administered between about 12 hours and about 24 hours apart. The CDC7 inhibitor and CDK8 (and/or CCNC) inhibitor may be administered between about 18 hours and about 30 hours apart. The CDC7 inhibitor and CDK8 (and/or CCNC) inhibitor may be administered about 24 hours apart.

Where separate administration occurs, the CDC7 inhibitor may be administered before the CDK8 (and/or CCNC) inhibitor. Alternatively, where separate administration occurs, the CDK8 (and/or CCNC) inhibitor may be administered before the CDC7 inhibitor.

The CDC7 inhibitor and CDK8 (and/or CCNC) inhibitor may be provided in a composition together or provided in separate compositions. The CDC7 inhibitor and CDK8 (and/or CCNC) inhibitor may optionally be administered in combination with another therapeutically active agent. Other therapeutically active agents may comprise other immune adjuvants such as lymphokines and cytokines. Examples include interferons such as alpha, beta, and gamma interferon, interleukins such as IL-2, Il-4, IL-6, IL-13 et al., colony stimulating factors, TNFs, and the like. Other therapeutically active agents may comprise other antitumor agents such as chemotherapeutics and cytotoxins commonly used for treating cancer, agents that inhibit angiogenesis, and the like. These additional therapeutic agents may be administered separately or in combination. These additional therapeutic agents may be co-formulated with the CDC7 inhibitor and/or CDK8 (and/or CCNC) inhibitor.

The CDC7 inhibitor and CDK8 (and/or CCNC) inhibitor can be administered locally or systemically by any method known in the art including but not limited to intramuscular, intravenous, intradermal, subcutaneous, intraperitoneal, intranasal, oral or other mucosal routes. Additional routes include intracranial (for example intracisternal, or intraventricular), intraorbital, ophthalmic, intracapsular and intraspinal administration. It is envisaged that injections will be the primary route for therapeutic administration of the compositions although delivery through a catheter or other surgical tubing is also used. Some suitable routes of administration include intravenous, subcutaneous, intraperitoneal and intramuscular administration. Liquid formulations may be utilised after reconstitution from powder formulations.

The CDC7 inhibitor and CDK8 (and/or CCNC) inhibitor of the present description will usually be administered in the form of a pharmaceutical composition, which may comprise at least one component in addition to the CDC7 inhibitor and CDK8 (and/or CCNC) inhibitor. The CDC7 inhibitor and CDK8 (and/or CCNC) inhibitor can be administered in a suitable, nontoxic pharmaceutical carrier, or can be formulated in microcapsules or a sustained release implant. The CDC7 inhibitor and CDK8 (and/or CCNC) inhibitor can be administered multiple times, if desired. The appropriate route, formulation, and immunization schedule can be determined by one skilled in the art.

The CDC7 inhibitor and CDK8 (and/or CCNC) inhibitor can be administered with a physiologically acceptable carrier such as physiological saline. The composition may also include another carrier or excipient such as buffers, such as citrate, phosphate, acetate, and bicarbonate, amino acids, urea, alcohols, ascorbic acid, phospholipids, proteins such as serum albumin, ethylenediamine tetraacetic acid, sodium chloride or other salts, liposomes, mannitol, sorbitol, glycerol and the like. The CDC7 inhibitor and CDK8 (and/or CCNC) inhibitor can be formulated in various ways, according to the corresponding route of administration.

The administration may be a therapeutically effective amount.

The CDC7 inhibitor and CDK8 (and/or CCNC) inhibitor agent, or otherwise compositions of the description, are preferably administered to an individual in a "therapeutically effective amount", this being sufficient to show benefit to the individual. The actual amount administered, and rate and time-course of administration, will depend on the nature and severity of what is being treated. Prescription of treatment, e.g., decisions on dosage etc., is within the responsibility of general practitioners and other medical doctors, and typically takes account of the disorder to be treated, the condition of the individual patient, the site of delivery, the method of administration and other factors known to practitioners. The compositions of the description are particularly relevant to the treatment of existing tumours, especially cancer, and in the prevention of the recurrence of such conditions after initial treatment or surgery. Examples of the techniques and protocols mentioned above can be found in Remington's Pharmaceutical Sciences, 16th edition, Oslo, A. (ed), 1980 (Remington and Osol, 1980).

The optimal dose can be determined by physicians based on a number of parameters including, for example, age, sex, weight, severity of the condition being treated, the active ingredient being administered and the route of administration.

The combination of the CDC7 inhibitor and CDK8 (and/or CCNC) inhibitor may facilitate a significant increase in survival of treated mice in a colorectal cancer COLO205 xenograft model, or alternatively a breast cancer MDA-MB-231, MDA-MB-468 or U-2-OS model.

The combination of the CDC7 inhibitor and CDK8 (and/or CCNC) inhibitor may be provided to patients/subjects that have failed to respond adequately to the standard care.

### The cancer

In one embodiment, the cancer to be treated or prevented may comprise a solid tumour malignancy. The cancer cell may comprise or consist of a cancer cell of a solid tumour. The solid tumour may comprise a sarcoma, carcinoma, or lymphoma. The cancer may comprise neuroblastoma or melanoma. Solid tumors that can be treated using the compositions and methods described herein may be selected from any one of the group of solid tumors of the breast, lung, colon, stomach, liver, kidney, ovary, and prostate; or combinations thereof. Tumors that can be treated in accordance with the description may be selected from breast carcinomas, lung carcinomas, prostate carcinomas, gastric carcinomas, esophageal carcinomas, colorectal carcinomas, liver carcinomas, ovarian carcinomas, vulval carcinomas, kidney carcinomas, cervical carcinomas, endometrial carcinoma, endometrial hyperplasia, endometriosis, choriocarcinoma, head and neck cancer, nasopharyngeal carcinoma, laryngeal carcinomas, hepatoblastoma, Kaposi's sarcoma, melanoma, skin carcinomas, hemangioma, cavernous hemangioma, hemangioblastoma, pancreatic carcinomas, retinoblastoma, astrocytoma, glioblastoma, Schwannoma, oligodendroglioma, medulloblastoma, neuroblastomas, sarcomas include fibrosarcomas, rhabdomyosarcoma, osteogenic sarcoma, leiomyosarcomas, urinary tract carcinomas, thyroid carcinomas, Wilm's tumor, brain tumors, renal cell carcinomas, abnormal vascular proliferation associated with phakomatoses, and edema (such as that associated with brain tumors); or combinations thereof. The cancer cell may comprise a cell of any one of the above solid tumours.

Additionally or alternatively, the cancer to be treated or prevented may be a blood cancer, such as leukemia, lymphoma or myeloma.

In one embodiment, the cancer to be treated or prevented is osteosarcoma or epithelial adenocarcinoma.

### Other descriptions

Also described is a CDC7 inhibitor in combination with a CDK8 and/or CCNC inhibitor for use in the treatment or prevention of cancer in a subject.

According to a second aspect of the present invention, there is provided a composition according to claim 5 herein.

Also described is a composition comprising a CDC7 inhibitor and a CDK8 and/or CCNC inhibitor.

According to a third aspect of the present invention, there is provided a kit according to claim 6 herein.

Also described is a kit for treatment or prevention of cancer in a subject, the kit comprising:
i) a CDC7 inhibitor; and
ii) a CDK8 and/or CCNC inhibitor.

Also described is a composition or kit according to the description for use as a medicament.

Also described is a composition according to the description , or kit according to the description , for use in the treatment or prevention of cancer in a subject.

According to a fourth aspect of the present invention, there is provided a method according to claim 12 herein.

According to a fifth aspect of the present invention, there is provided a composition according to claim 13 herein.

Also described is a method of treatment or prevention of cancer in a subject, wherein the cancer is determined or known to be deficient in functional CDK8 and/or CCNC expression, the method comprising the administration of a Cell Division Cycle 7 kinase inhibitor (CDC7i) to the subject.

According to a sixth aspect of the present invention, there is provided a CDC7i for the use according to claim 9 herein.

Also described is a Cell Division Cycle 7 kinase inhibitor (CDC7i) for use in a method of treatment or prevention of cancer in a subject, wherein the cancer is determined or known to be deficient in functional CDK8 and/or CNCC expression.

The description recognises that some subjects may have a cancer that is CDK8 deficient, which would then obviate the need for a CDK8i and/or CCNCi to be administered in combination with a CDC7i. The skilled person will recognise that cancers deficient in CDK8 and/or CNCC represent a sub-class of cancers that may be targeted for treatment more efficiently by single CDC7i therapy.

For example, a percentage of Diffuse large B cell lymphoma (DLBCL) is understood to be CDK8 deficient. Therefore, the method may comprise the treatment of DLBCL having CDK8 deficiency with a CDC7 inhibitor (CDC7i).

CDK8 deficiency has been implicated in some cases of Sarcoma (1.6 %; 4/255 cases); Lung Adenocarcinoma (1.1 % (6/566 cases); and Diffuse Large B-Cell Lymphoma (1.2 %; 1/48 cases). Therefore, the method may comprise the treatment of such cancers having CDK8 deficiency with a CDC7 inhibitor (CDC7i).

CCNC deficiency has been implicated in some cases of Diffuse Large B-Cell Lymphomas (10.42 %; 5/48 cases); Prostate Adenocarcinoma (8.7 %; 43/494 cases), Uveal Melanoma (6.3 %; 5/80 cases); Liver Hepatocellular Carcinoma (2.4 %; 9/372 cases); Stomach Adenocarcinoma (1.6 %; 7/440 cases); and Bladder Urothelial Carcinoma (1.5 %; 6/411 cases). Therefore, the method may comprise the treatment of such cancers having CCNC deficiency with a CDC7 inhibitor (CDC7i).

CDK8 deficiency is understood to mean that the cells, such as the cancer cells, do not express functional CDK8, or the CDK8 expression is significantly lower than average expression levels in normal cells (i.e. non-CDK8-deficient cells), such as at least 3-fold, 5-fold, 10-fold, 50-fold or 100-fold lower.

CCNC deficiency is understood to mean that the cells, such as the cancer cells, do not express functional CCNC, or the CCNC expression is significantly lower than average expression levels in normal cells (i.e. non- CCNC -deficient cells), such as at least 3-fold, 5-fold, 10-fold, 50-fold or 100-fold lower.

### Biomarkers

It is advantageously found that tumors defective in either CDK8 and/or CCNC can be particularly sensitive to CDC7i-basesd chemotherapy.

Therefore, also described is the use of CDK8 and/or CCNC as biomarkers for predicting the efficacy of CDC7i therapy for cancer of a subject, wherein the reduction or absence of expression of functional CDK8 and/or CCNC in a subject is indicative of an increased efficacy of the CDC7i therapy for cancer.

Also described is a method of predicting the efficacy of a CDC7i therapy for cancer in a subject, the method comprising determining the levels of expression of functional CDK8 and/or CCNC in the subject, or obtaining results of such a determination,
wherein a decreased level of expression of CDK8 and/or CCNC in the subject is indicative of a more effective CDC7i therapy for the cancer in the subject.

Also described is a method of selecting a subject for treatment for cancer with a CDC7i, the method comprising determining the levels of expression of functional CDK8 and/or CCNC in the subject, or obtaining results of such a determination,
wherein the subject is selected for CDC7i treatment if they have a decreased level of expression of CDK8 and/or CCNC.

The methods or uses described may further comprise the treatment of the subject with a CDC7i, for example by administration of a CDC7i to the subject.

Also described is a method of treatment of a subject for cancer, the method comprising:
- determining the levels of expression of functional CDK8 and/or CCNC in the subject, or
- having obtained results of the levels of expression of functional CDK8 and/or CCNC in the subject; and
- administering CDC7i treatment if the subject has a decreased level of expression of CDK8 and/or CCNC.

The increased efficacy may be relative to cancer subjects having normal or higher levels of expression of functional CDK8 and/or CCNC. The increased efficacy may be a statistically significant increase in efficacy. The decreased level of expression of CDK8 and/or CCNC may be relative to a reference level. The reference level may be an average level of expression in a general population, or an average level in a population of subjects having cancer, preferably the same cancer type and/or stage.

The reduction or absence of expression of functional CDK8 and/or CCNC in a subject may be in a selected tissue, such as tumour tissue or cells. For example the tumour tissue or cells of the subject may be determined for their expression of functional CDK8 and/or CCNC.

The reduction/decreased level of expression of functional CDK8 and/or CCNC may be a 100% decrease. In another embodiment, the decreased level of expression of CDK8 and/or CCNC may be a 50%, 80%, 90%, 95% or 99% decrease.

The CDC7i may be administered and/or formulated as described herein. The CDC7i may be a The CDC7i described herein.

### Definitions

The term "inhibitor" is understood to refer to an agent that is capable of preventing or reducing the activity of a target molecule, or a biological pathway. The inhibition may be complete, i.e. 100% inhibition, or the inhibition may be partial. For example, the inhibition may inhibit up to 40%, 50%, 60%, 70%, 80%, 90%, 95% or 99% of the original activity of the target (i.e. relative to the activity when not in the presence of the inhibitor). The inhibition may be sufficient to effect a statistically significant reduction in activity of the target. In one embodiment, the level of inhibition is sufficient to cause a therapeutic effect.

The term "subject" means all animals including humans. Examples of subjects include humans, cows, dogs, cats, goats, sheep, and pigs. The term "patient" means a subject having a disorder in need of treatment.

A 'therapeutically effective amount', or 'effective amount', or 'therapeutically effective', as used herein, refers to that amount which provides a therapeutic effect for a given condition and administration regimen. This is a predetermined quantity of active material calculated to produce a desired therapeutic effect in association with the required additive and diluent, i.e. a carrier or administration vehicle. Further, it is intended to mean an amount sufficient to reduce and most preferably prevent, a clinically significant deficit in the activity, function and response of the host. Alternatively, a therapeutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluent. In the methods and use for manufacture of compositions of the description, a therapeutically effective amount of the active component is provided. A therapeutically effective amount can be determined by the ordinary skilled medical or veterinary worker based on patient characteristics, such as age, weight, sex, condition, complications, other diseases, etc., as is well known in the art.

The term "functional" is understood to refer to the normal function of a given biological molecule such as CDC7, CDK8 or CCNC. A loss of function may be complete, such that it cannot carry out its normal ligand binding and/or reaction, or it may have a significant impairment in activity, such as a lower ability to bind its ligand or a lower rate of reaction. A loss of "function" or "non-functional molecule" may be a loss of the activity of the molecule, or a lack of expression of the molecule.

The term "functional expression" is understood to refer to the expression of a functional molecule having its inherent activity.

The skilled person will appreciate that preferred features of any one embodiment and/or description may be applied to all other embodiments and/or descriptions.

Examples embodying an aspect of the invention will now be described with reference to the following figures:

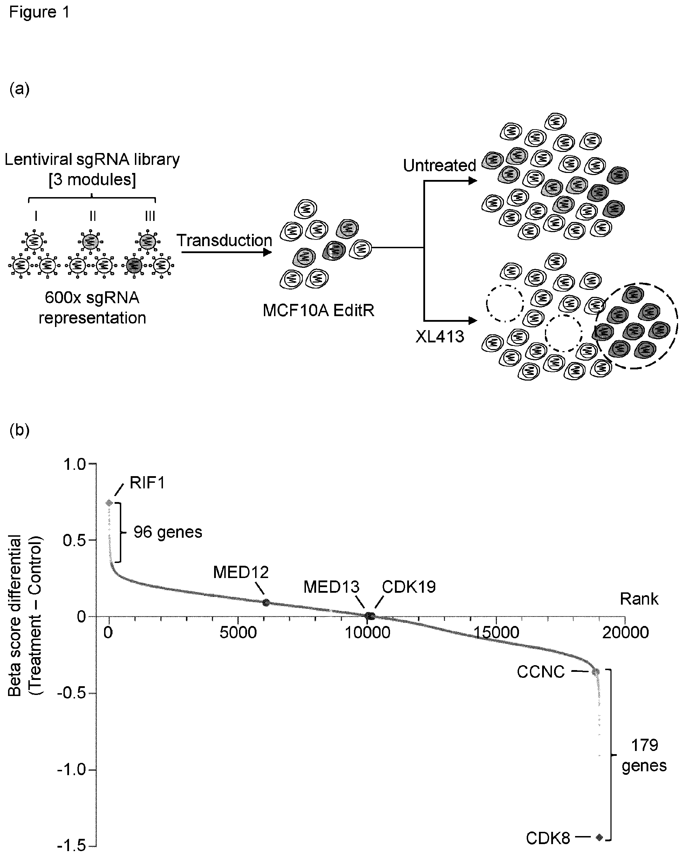

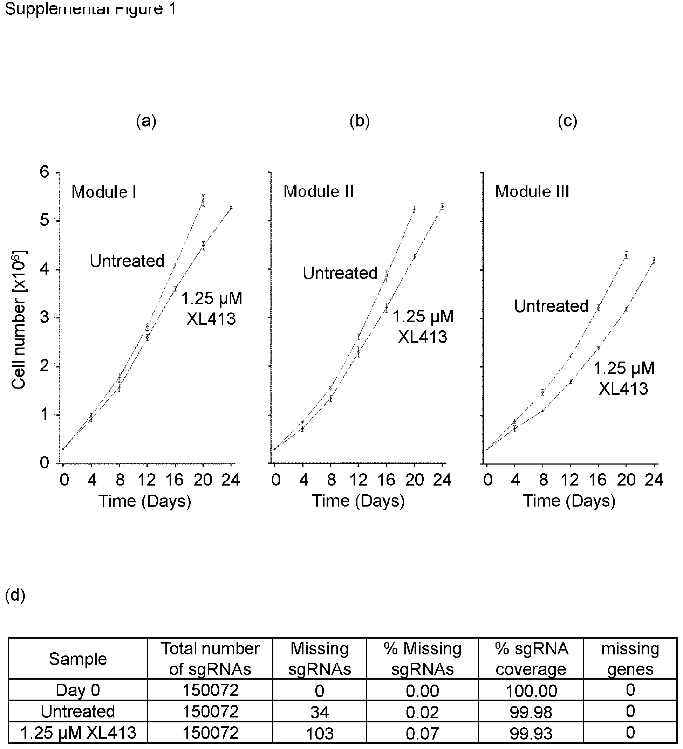

Figure 1: **Genome-wide CRISPR/Cas9 screen identifies genes that modulate proliferation in response to a CDC7 inhibitor**
   (a) Representation of the genome-wide CRISP/Cas9 screen workflow used to identify genes, which upon gene editing, either enhance or suppress the anti-proliferative effects of XL413 indicated by grey dot-dash or black broken circles, respectively.
   (b) Ranked plot of the differential between the XL413-treated and control beta scores obtained from MAGeCKFlute analysis. Significant gene outliers were identified using thresholds of +/- 2 standard deviations from the mean differential. Lighter grey dots represent candidate genes, which upon gene editing, either significantly enhance (179 genes) or suppress (96 genes) the anti-proliferative effects of XL413, respectively. Genes of interest have been highlighted and named.

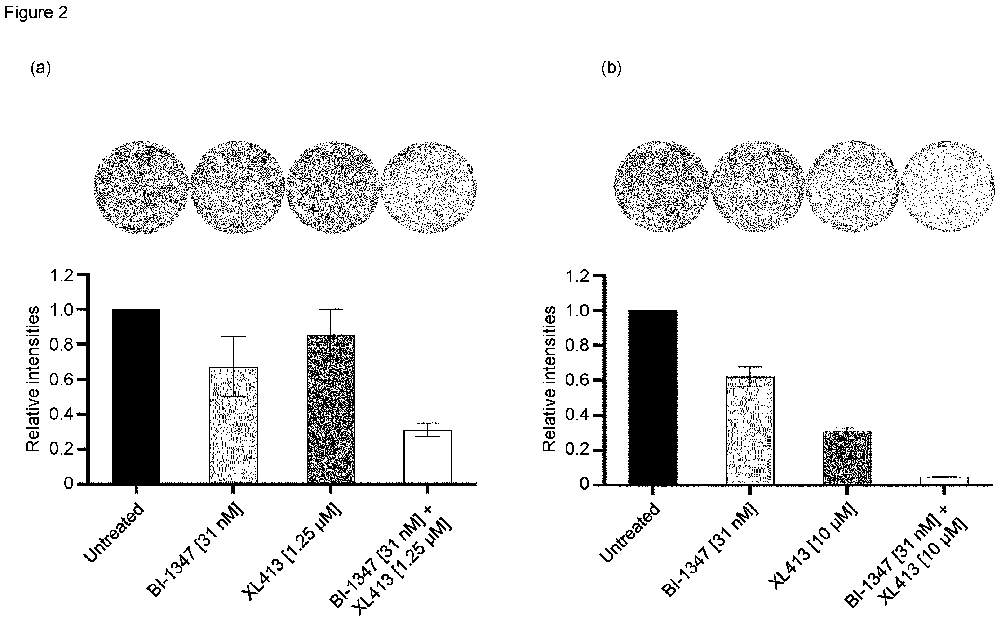

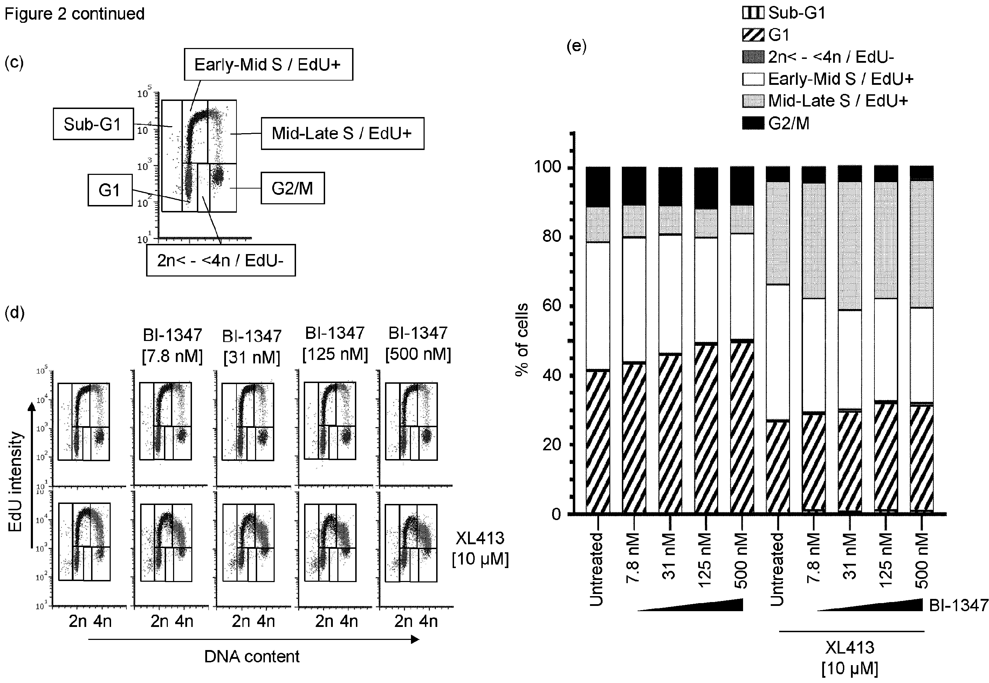

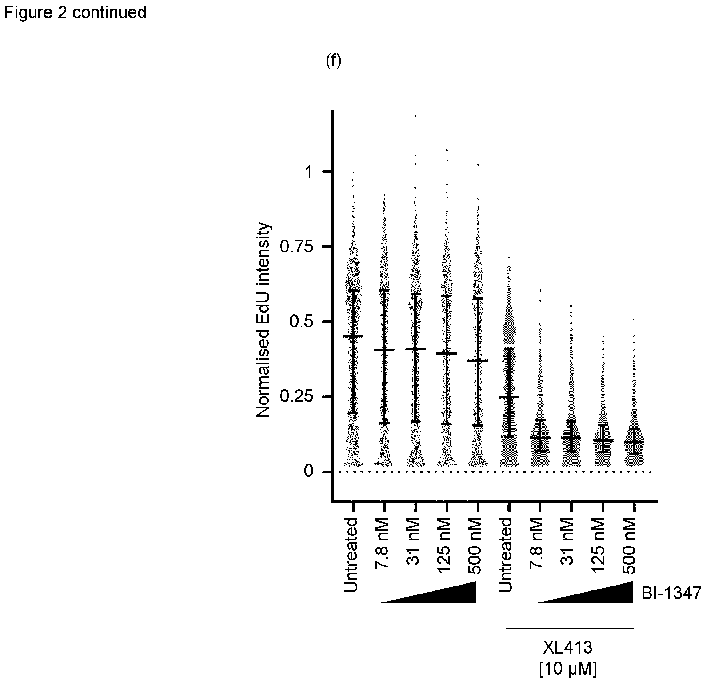

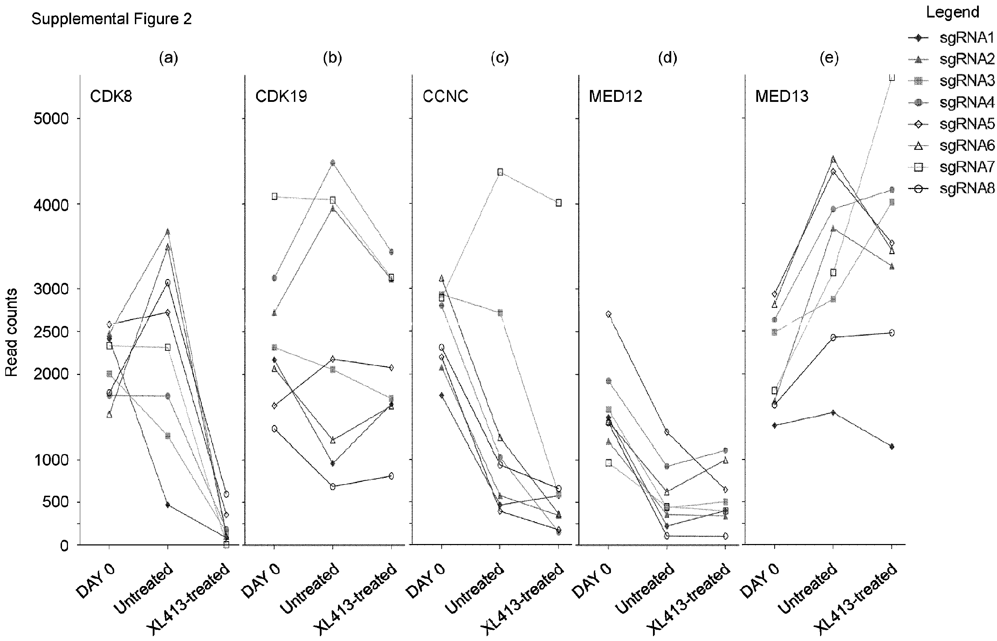

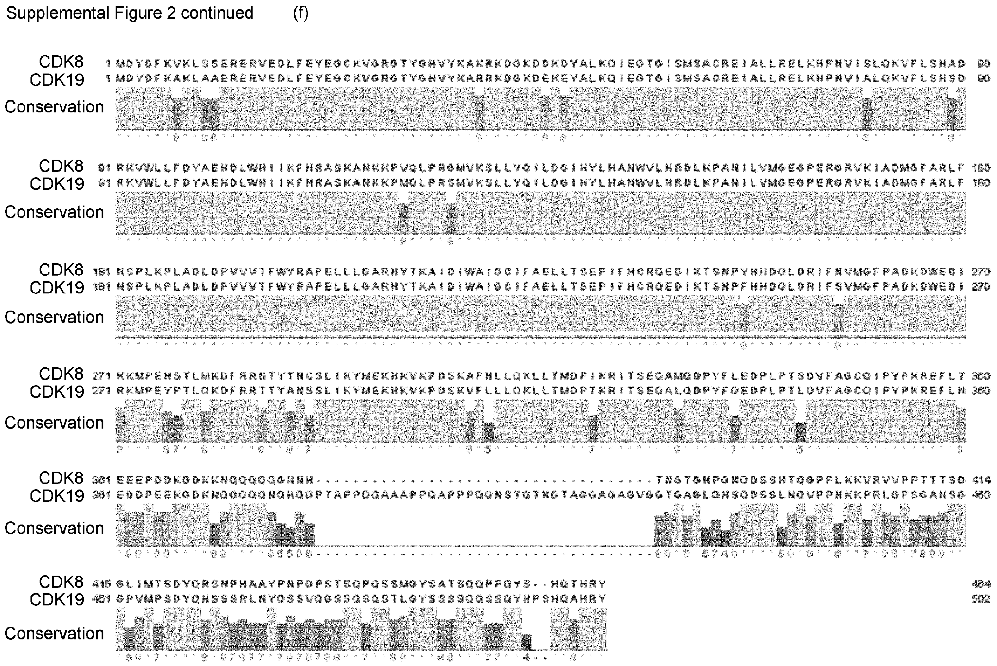

Figure 2: **CDK8 and CDC7 inhibitors synergise to restrict cell proliferation and DNA synthesis**
   (a and b) MCF10A cells were plated at equal low density and then either mock treated or treated with 31 nM BI-1347, in the presence or absence of XL413 at a doses of (a) 1.25 µM or (b) 10 µM over a 7 day period. Representative images of crystal violet-stained cells are shown alongside quantitation of crystal violet intensity normalised to the untreated control. Graph shows the mean ± SD of three independent experiments.
   (c) A typical profile obtained by flow cytometry of MCF10A cells labelled with EdU and DAPI that indicates the gating applied in order to determine the percentage of cells with sub-G1 DNA content (sub-G1), G1 cells (G1), replicating early-mid S phase cells (Early-Mid S / EdU+), replicating mid-late S phase cells (Mid-Late S / EdU+), cells with S phase DNA content but not replicating (2n< & <4n / EdU-), and cells either in G2 or mitosis (G2/M).(d-f) MCF10A cells were mock treated or treated with 10 µM XL413, in the presence or absence of increasing concentrations of the CDK8 inhibitor BI-1347, for 24 hours. (d) Cells were labelled with EdU for last 30 minutes and DNA synthesis, DNA content and cell cycle distribution were assessed by flow cytometry and analysed using the gating strategy described in above (Figure 2c). (e) Distribution of cells in the cell cycle. (f) Fluorescence intensity, proportional to EdU incorporation, in individual S phase cells. Long and short horizontal black lines indicate the median and the interquartile range, respectively, extending from the 25^{th} to the 75^{th} percentile for ~3,810 cells for each condition. Data is from a single experiment.

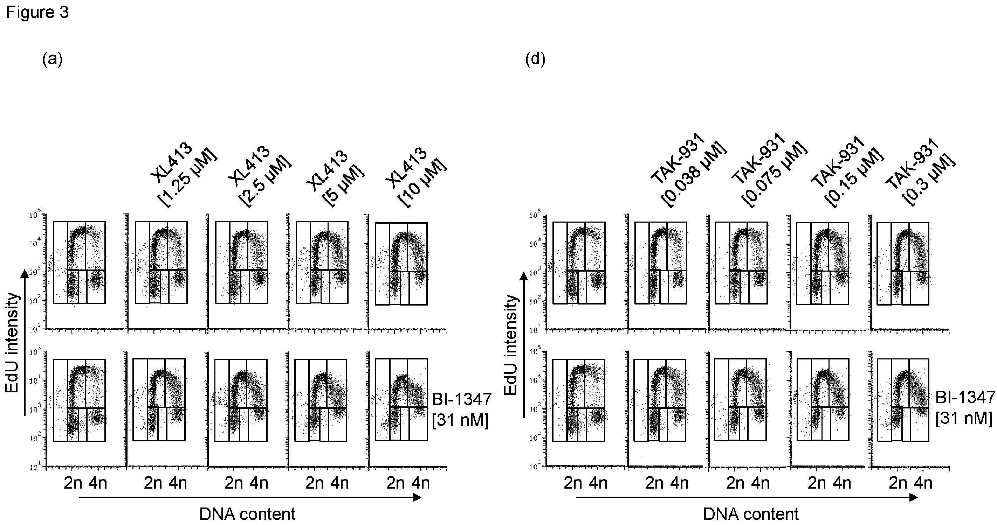

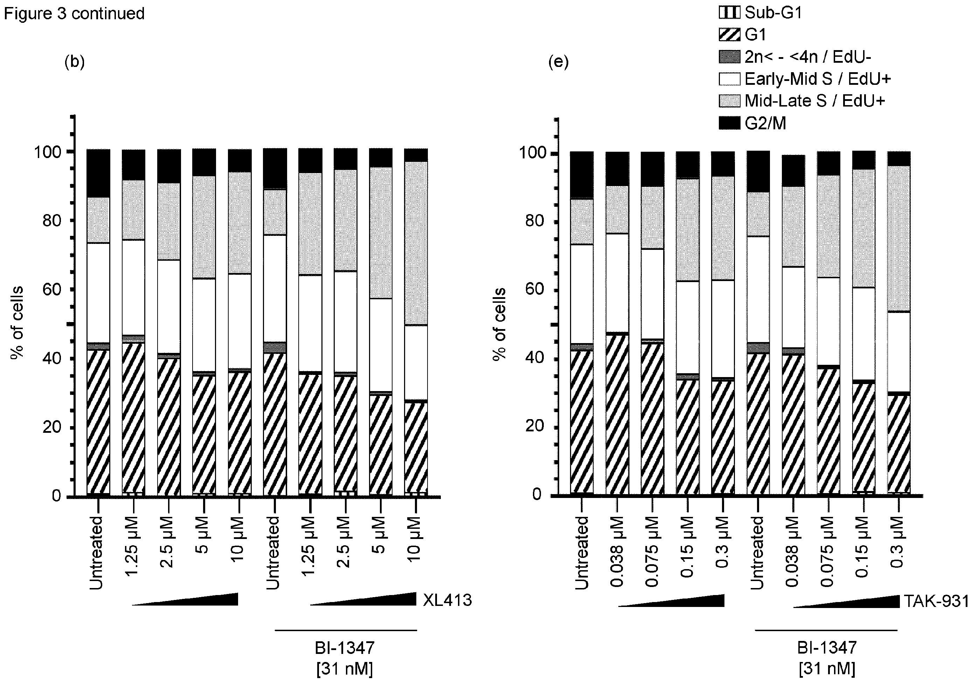

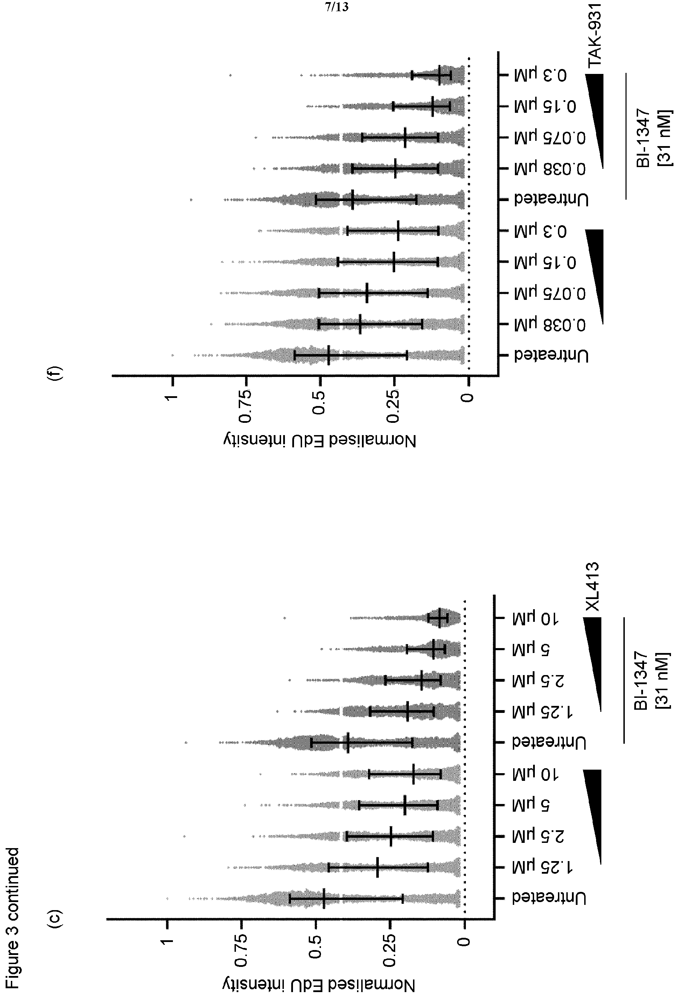

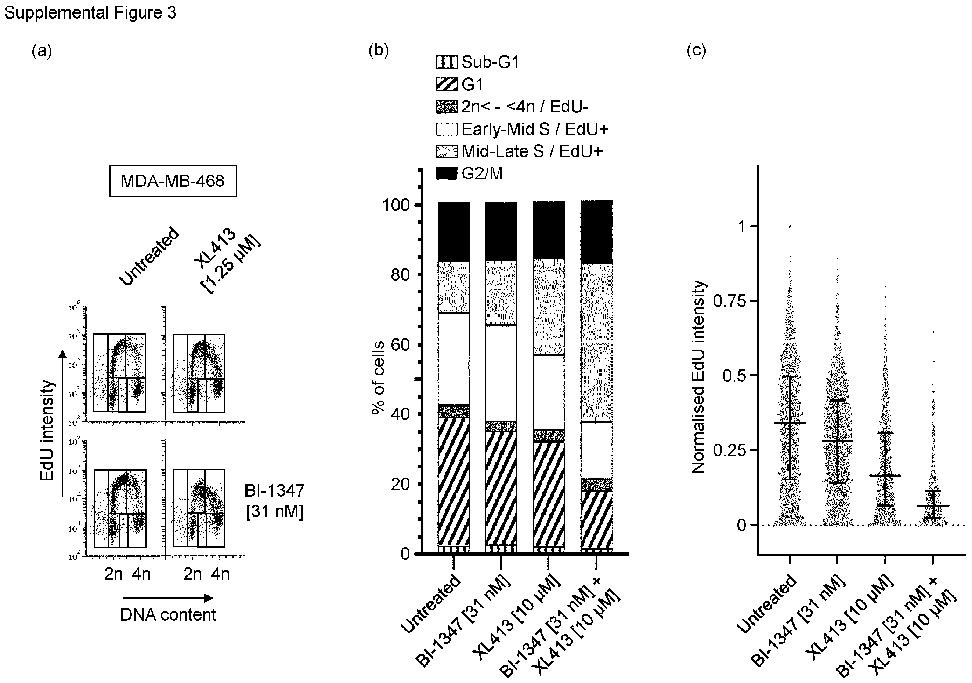

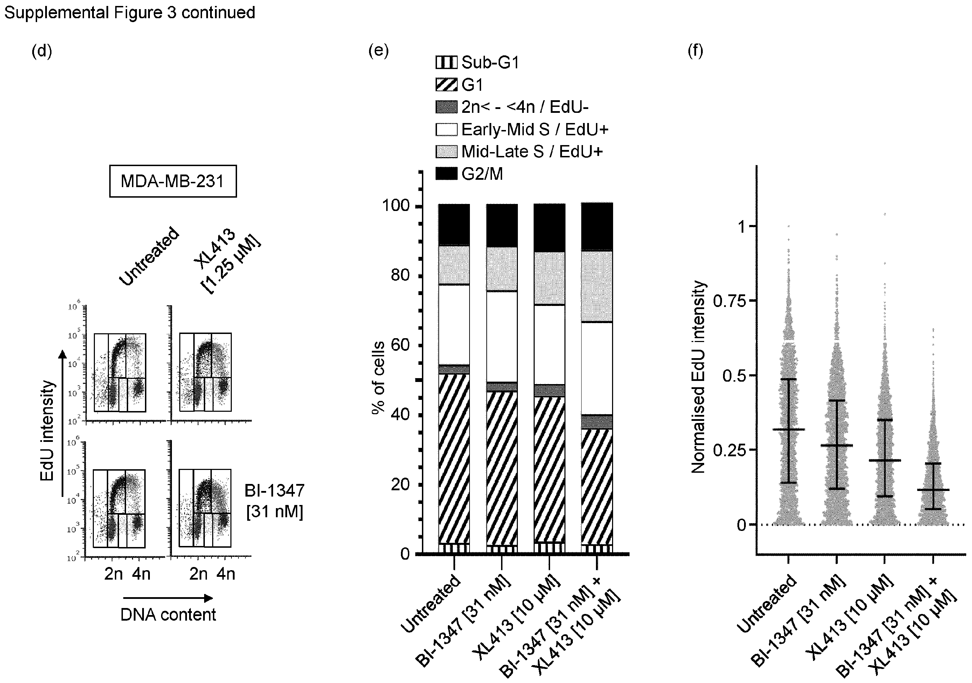

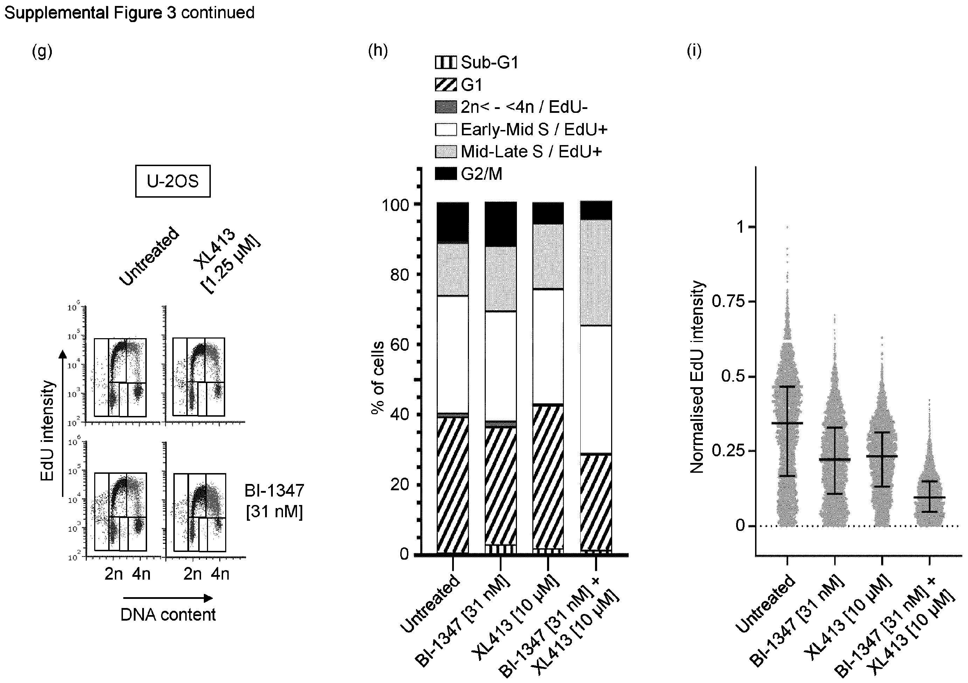

Figure 3: **CDK8 activity is required for efficient DNA synthesis under conditions of limited CDC7 kinase activity** (a - c) MCF10A cells were mock treated or treated with 31 nM BI-1347, in the presence or absence of increasing concentrations of the CDC7 inhibitors, XL413 (a-c) and TAK-931 (d-f), for 24 hours. (a and d) Cells were labelled with EdU for last 30 minutes and DNA synthesis, DNA content and cell cycle distribution were assessed by flow cytometry and analysed using the gating strategy described in Figure 2c.c (b and e) distribution of cells in the cell cycle. (c and f) Fluorescence intensity, proportional to EdU incorporation, in individual S phase cells. Long and short horizontal black lines indicate the median and the interquartile range, respectively, extending from the 25^{th} to the 75^{th} percentile for ~4,190 cells for each condition. Data is from a single experiment.

### SUPPLEMENTAL FIGURE LEGENDS

Supplemental Figure 1: **CRISPR/Cas9 screen: Cell proliferation curves, summary of sgRNA coverage, and genes of interest identified**
   (a - c) Proliferation curves of MCF10A EditR cells expressing sgRNAs from CRISPR library modules I, II and III in the absence (untreated) or presence of 1.25 µM XL413. Data is from three independent counts of each sample ± standard deviation (SD). (d) DNA extracted from samples of the CRISPR/Cas9 screen, which were harvested on day 0 and after 20 or 24 days of growth in the absence (untreated) or presence of 1.25 µM XL413, respectively, was analysed by next generation sequencing. Sequence data was mapped to a CELLECTA genome-wide sgRNA reference library using the MAGeCKFlute pipeline. (d) The table summarises the total and missing number of sgRNAs, the calculated % sgRNA coverage and the number of missing genes.
Supplemental Figure 2: **Relative changes in abundance of sgRNAs that target genes of the CDK8/CDK19-kinase module in the CRISPR/Cas9 screen and protein sequence alignment of CDK8/CDK19 paralogs**
   (a - e) Dot plots of normalised sgRNA read counts for day 0, day 20 untreated and day 24 XL413-treated samples for genes that express the core components of the CDK8/CDK19-kinase module of the mediator complex. Dots are connected by lines as a visual aid to track abundance changes of sgRNAs across different samples. (f) Protein sequences for CDK8 (NP001251, amino acids: 1 to 464) and CDK19 (NP055891, amino acids: 1-502) were aligned using Clustal Omega, showing 384 identical sites with an overall pairwise identity of 75 %. The degree of conservation of aligned amino acid residues in each column is summarised as a bar graph.
Supplemental Figure 3: **Characteristic phenotypes associated with inhibition of CDC7 and CDK8 are observed in multiple cell lines**
   A panel of different cell lines, including: MDA-MB-468 (a - c); MDA-MB-231 (d - f); and U-2 OS (g - i), were mock treated or treated with 1.25 µM XL413 and/or 31 nM BI-1347BI-1347 for 24 hours. Cells were labelled with EdU for last 30 minutes and DNA synthesis, DNA content and cell cycle distribution were assessed by flow cytometry and analysed using the gating strategy described in Figure 2c. (b, e and h) distribution of cells in the cell cycle. (c, f and i) Fluorescence intensity, proportional to EdU incorporation, in individual S phase cells. Long and short horizontal black lines indicate the median and the interquartile range, respectively, extending from the 25^{th} to the 75^{th} percentile for ~ 3,922 (MDA-MB-468), ~ 3,291 (MDA-MB-231), and ~4,628 (U-2 OS) cells for each condition. Data is from a representative experiment from 3 biological repeats.

### Example 1 - Enhancement of the anti-proliferative activity of CDC7 inhibitors by CDK8 inhibition

### INTRODUCTION

Eukaryotic DNA replication initiates at multiple origins according to a well-defined spatiotemporal program, with origins being activated in early, mid-, or late S phase, each one giving rise to two replication forks (Gilbert et al., 2010). Origin activation is orchestrated by the coordinate action of Cyclin-dependent kinases (CDKs) and CDC7 kinase, also known as DDK (DBF4-dependent kinase). In this endeavour CDC7 is known, by phosphorylating several subunits of the MCM complex (Gilbert et al., 2010; Masai et al., 2006; Sheu and Stillman, 2006, 2010), to support formation and activation of the Cdc45-MCM-GINS (CMG) helicase complex (Labib, 2010).

Many small-molecule ATP-competitive CDC7 inhibitors (CDC7is) have been developed as the focus of multiple drug discovery programs in oncology as an alternative strategy to restrain DNA replication by limiting origin firing instead of directly blocking fork progression (Montagnoli et al., 2010). Inhibition of CDC7 in cells leads mitotic abnormalities as well as replication stress, with decreased origin activation, delayed S-phase progression and activation of the S-phase checkpoint (Iwai et al., 2019; Rainey et al., 2020). The latter involves ETAA1-dependent activation of ATR to further suppress origin activation and prevent premature entry and catastrophic progression through an aberrant mitosis with under-replicated DNA (Rainey et al., 2020).

Several CDC7is have shown potent anti-proliferative activity *in vitro,* and in several cases, excellent anti-tumor activity in preclinical models in vivo (Iwai et al., 2019, 2021; Montagnoli et al., 2008), with the most advanced compounds currently in early open clinical trials, including: LY3143921 (Clinicaltrials.gov ID: NCT03096054), and TQB3824 (NCT05028218), or in trials that have been completed or terminated: BMS-863233 (NCT00838890 and NCT00886782), NMS-1116354 (NCT01016327 and NCT01092052) and TAK-931 (NCT02699749).

To better guide the clinical development a recent study combined the CDC7i, TAK-931 with different chemotherapeutic drugs, and showed that the combination of TAK-931 with several DNA-damaging agents synergized to produce the strongest anti-proliferative effects *in vitro.* The efficacy of combination therapies was confirmed using preclinical primary-derived xenograft models (Iwai et al., 2021). However, in order to properly position CDC7 inhibitors in the clinic we require (1) a better understand of how tumour cells respond to this new class of drugs and (2) how to increase their efficacy without affecting their tolerability so that the therapeutic window is improved.

While CDC7 is an essential gene in almost every cell line tested thus far (Cowley et al., 2014; McFarland et al., 2018; Meyers et al., 2017; Rainey et al., 2017; Tsherniak et al., 2017), it is now clear that different cancer cells have a wide range of sensitivity to either partial or transient CDC7 inhibition (Iwai et al., 2019; McDonald et al., 2017; Sasi et al., 2014). This suggests that multiple factors may determine how cells respond to decreased levels of CDC7 activity and that the response to limited origin firing is genetically controlled.

To understand how the problems in DNA replication arising from CDC7 inhibition are dealt with we performed a genome-wide CRISPR/Cas9 chemogenetic screen to identify genes, that upon loss, could sensitize to the anti-proliferative effects of CDC7i. As our top hit we identified CDK8 and its cognate Cyclin, Cyclin C (CCNC), as a significant hit.

CDK8 is a ubiquitously expressed nuclear Ser/Thr kinases, that is a component of the "Mediator-CDK8 module", and is traditionally classified as a "transcriptional" rather than a "cell cycle" CDK (Dannappel et al., 2019; Galbraith et al., 2010). The Mediator is a large multi-subunit complex, consisting of a "head", "middle", and "tail" at the core of its structure, which is essential for its function in transcription by bridging transcriptional elements, promoters, chromatin modifiers and transcription factors (TFs) to RNA polymerase II (Fant and Taatjes, 2019; Harper and Taatjes, 2018). The "CDK8 module" is a 4 subunit regulatory subcomplex in which Cyclin C (CCNC) and MED12, activate CDK8 kinase function, and MED13, enables their interaction with the Mediator (Dannappel et al., 2019; Fant and Taatjes, 2019; Menzl et al., 2019). In mammals a paralog of CDK8, namely CDK19, exists and has high sequence similarity to CDK8. CDK19 in a mutually exclusive manner with CDK8, can form an analogous "CDK19 module" (Tsutsui et al., 2008). To further complicate matters paralogs of MED12 and MED13 also exists, termed MED12L (MED12-like) and MED13L (MED13-like) respectively.

CDK8, primarily regulates cellular function through phosphorylation of a large number of proteins including: Mediator components, TFs; chromatin remodelers, and RNA polymerase II, in addition, to non-mediator associated proteins (Poss et al., 2016). CDK8 activity is not required for bulk transcription, but it regulates the expression of subsets of genes in cell type- and context-specific manner. This uniquely highlights CDK8 as mediators of transcriptional reprogramming, rather than regulators of global transcription, like CDK7 and CDK9 (Chen et al., 2019; Fant and Taatjes, 2019; Nemet et al., 2014).

CDK8 was first described as a proto-oncogene in colorectal cancer (CRC), where high expression levels negatively correlated with poor patient survival and were associated with advance stage disease progression (Firestein et al., 2008, 2010; Seo et al., 2010). Interestingly, downregulation of CDK8 reduced proliferation of CRC cells *in vitro* and in xenograft models with CDK8 amplification, highlighting the therapeutic potential of targeting CDK8 (Adler et al., 2012; Firestein et al., 2008; Seo et al., 2010). As a result several CDK8 inhibitors have now been developed as targeted agents and because of the similarity between CDK8 and CDK19 active site most of these compounds can be considered as dual CDK8/19 inhibitors (Fant and Taatjes, 2019; Philip et al., 2018; Xi et al., 2019). Some of these have shown cancer type-specific therapeutic effects in *in vivo* colorectal (Liang et al., 2018), breast (McDermott et al., 2017), leukemia (Pelish et al., 2015; Rzymski et al., 2017), prostate (Nakamura et al., 2018) and lung (Porter et al., 2012) models. Inhibition of CDK8 signalling was also shown to modulate the cytotoxic effects of NK cells, promoting tumour surveillance and increasing survival in mouse melanoma, leukemia and metastasising breast cancer models (Hofmann et al., 2020; Putz et al., 2013).

Taken together all these observations indicate that the antitumor activity of CDK8 inhibitors could be mediated by both cell-intrinsic mechanisms, leading to reduced tumour cell proliferation and survival, as well as cell-extrinsic mechanism mediated by the host immune system and tumor microenvironment.

Thus, CDK8 inhibition is a potentially promising therapeutic approach, however, as in the case of CDC7is, the development of CDK8 based therapy has obstacles to overcome. For example tolerability issues have been observed with some inhibitors (Chen et al., 2019) and as a result only select inhibitors, RVU120 and BCD-115, have progressed to clinical trials targeting acute myeloid leukemia (ClinicalTrials.gov Identifier: NCT04021368) and estrogen-receptor positive breast cancers (ClinicalTrials.gov Identifier: NCT03065010), respectively. Due to the cell-type specific regulation of gene expression by CDK8, the best indication for a CDK8i based therapy has not yet been identified and maybe limited to only a few cancer types/subtypes. Finally, all CDK8 inhibitors identified so far also target CDK19, and developing monospecific CDK8 inhibitors could be a challenge due to the high sequence similarity shared between CDK8 and CDK19 (Philip et al., 2018; Tsutsui et al., 2008).

While described as transcriptional mediators, a recent paper has shown that CDK8/CDK19-CCNC can bind to the human homolog of yeast sld7 protein, MTBP, an essential DNA replication origin firing factor (Boos et al., 2013). This association occurs independently from MED12 and MED13, suggesting this could represent a role independent from the Mediator complex and transcription (Köhler et al., 2019). This interaction is mediated by a metazoan specific binding region in MTBP that is important for efficient DNA synthesis, preventing replication stress and ensuring complete genome duplication to support a normal mitosis (Köhler et al., 2019). However, while CDK8/CDK19-CCNC can bind and phosphorylate MTBP *in vitro,* CDK8 dependent phosphorylation of MTBP in living cells has yet not been detected nor studied (Ferreira et al., 2021; Köhler et al., 2019).

In this work we show that a dual-specificity inhibitor of CDK8/CDK19 only very subtly reduces the rate of DNA synthesis, but in combination with the CDC7 inhibitors, XL413 and TAK-931, dramatically blocks DNA synthesis and cell proliferation in a variety of human cell lines. We propose that inhibition of CDK8 can strongly potentiates the broad antitumor activity of CDC7 inhibition and, by doing so, it can increase the spectrum of tumour responding to a CDK8i and CDC7i combination therapy compared to monotherapy.

### RESULTS

### A genome-wide CRIPSR/Cas9 loss of function screen identifies genes that affect proliferation rate in response to a CDC7 inhibitor

In order to identify genes that change the rate of proliferation when DNA synthesis is mildly limited by CDC7 inhibition, we performed a CRISPR/Cas9 genome-wide knockout screen as outlined in Figure 1a. Briefly, MCF10A EditR cells that stably express Cas9 were transduced with a lentiviral-sgRNA library targeting approximately 19,000 coding genes and then were either untreated or treated with 1.25 µM of selective ATP competitive CDC7 inhibitor, XL413 (Koltun et al., 2012). Cell proliferation was monitored over the course of the screen and, consistent with previous findings (Rainey et al., 2017), this concentration of XL413 only mildly suppressed cell proliferation in this cell line (Supplemental Figure 1a-c). Next generation sequencing was then used to determine sgRNA abundance at day 0, day 20 untreated and day 24 XL413 treated cells. Preliminary analysis showed that the sgRNA library representation at the beginning of the experiment and at the end-points was nearly complete and that full coverage of all protein encoding genes was achieved in the screen (Supplemental Figure 1d). CRISPR/Cas9-mediated loss of function of genes involved in supporting proliferation in untreated cells or in response to CDC7 inhibition is expected to result in a decreased relative abundance of the corresponding sgRNAs in the cell population. By analysing the data from the two experimental branches we identified ~179 genes that, when knocked out, potentially decreased the proliferation rate specifically in CDC7i treated cells (Figure 1b). When the genes were ranked according to the magnitude of the effect, CDK8, a gene that expresses a component of the CDK8/CDK19-CCNC-MED 12-MED13 mediator complex kinase-module (Dannappel et al., 2019; Fant and Taatjes, 2019; Menzl et al., 2019), came out as the top hit (Figure 1b and Table 1, ranked 19012 out of 19012 genes).

**Table 1. The table summarises the beta scores, assigned by MAGeCK-MLE, and based on changes of sgRNA abundance from day 0 to day 20 or 24 in untreated or XL413-treated samples, respectively. Genes of interest are shown in rank order, by the differential beta score, alongside the number of sgRNAs used to target each gene, Uniprot identifier (Uniprot ID), protein name and functional annotation. Significant gene outliers were identified using thresholds of +/- 2 standard deviations from the mean differential of the beta score (Treatment - Control). (^{*1}) and (^{*2}) represent candidate gene hits, which upon editing, either significantly enhance or suppress the anti-proliferative effects of XL413, respectively.**

| **Rank** | **Gene** | **XL413 beta score** | **Untreated beta score** | **Differential beta score** | **Uniprot ID** | **Uniprot protein name and functional annotation** |
|---|---|---|---|---|---|---|
| 1 (^{*1}) | RIF1 | - 0.0162 | -0.7603 | 0.7442 | Q5UIP0 | Replication timing regulatory factor 1; Telomere-associated protein RIF1; Key regulator of TP53BP1 that plays a key role in the repair of double-strand DNA breaks (DSBs) in response to DNA damage: acts by promoting non-homologous end joining (NHEJ)-mediated repair of DSBs. |
| 6089 | MED12 | -0.8276 | -0.9204 | 0.0928 | Q93074 | Mediator of RNA polymerase II transcription subunit 12; Component of the Mediator complex, a coactivator involved in the regulated transcription of nearly all RNA polymerase II-dependent genes. Mediator functions as a bridge to convey information from gene-specific regulatory proteins to the basal RNA polymerase II transcription machinery. Mediator is recruited to promoters by direct interactions with regulatory proteins and serves as a scaffold for the assembly of a functional preinitiation complex with RNA polymerase II and the general transcription factors. |
| 10046 | MED 13 | 0.0939 | 0.0877 | 0.0062 | Q9UHV7 | Mediator of RNA polymerase II transcription subunit 13; Component of the Mediator complex, a coactivator involved in the regulated transcription of nearly all RNA polymerase II-dependent genes. |
| | | | | | | ***NB: Mediator function and recruitment as described for MED12.*** |
| 10184 | CDK19 | - 0.2393 | -0.2427 | 0.0034 | Q9BWU1 | Cyclin-dependent kinase 8/11; Cyclin dependent kinase 19; Belongs to the protein kinase superfamily. CMGC Ser/Thr protein kinase family. |
| 18866(^{*2}) | CCNC | - 1.0759 | -0.7157 | -0.3602 | P24862 | Cyclin-C; Component of the Mediator complex, a coactivator involved in regulated gene transcription of nearly genes. all RNA polymerase II-dependent genes. |
| | | | | | | ***recruitment NB: Mediator function and recruitment as described for MED12.*** |
| 19012(^{*2}) | CDK8 | - 1.6094 | -0.1703 | -1.4391 | P49336 | Cyclin-dependent kinase 8; Component of the Mediator complex, a coactivator involved in regulated gene transcription of nearly all RNA polymerase II-dependent genes. Phosphorylates the CTD (C-terminal domain) of the large subunit of RNA polymerase II (RNAp II), which may inhibit the formation of a transcription initiation complex. |
| | | | | | | ***NB: Mediator function and recruitment as described for MED12.*** |

Significantly, CCNC, the cognate cyclin of CDK8, was also required to support efficient proliferation specifically in response to CDC7i, while the CDK8 paralog, CDK19, and other components of the mediator complex kinase-module, MED12 and MED13, were not required. In addition, we also identified ~96 genes, which upon loss of function, could increase the rate of proliferation specifically in cells treated with low dose CDC7i. Importantly RIF1, a gene we have showed could reduce the requirement for CDC7 kinase when deleted in our previous CRISPR/Cas9 screen study (Rainey et al., 2020), was once again ranked as the top hit in this gene cohort and instils confidence in the results of this screen. (Figure 1b and Table 1, ranked 1 out of 19012 genes).

Interestingly CRISPR targeting of CDK8 showed significant and robust decreases in accumulation of multiple sgRNAs, but only under conditions of limited CDC7 activity (Supplemental Figure 2a). In contrast the targeting of the CDK8 paralog, CDK19, which shows 76% sequence identity (Supplemental Figure 2f), and which is also regulated by CCNC, does not strongly or reproducibly affect sgRNA abundance between the untreated and CDC7i treated branches (Supplemental Figure 2b). A similar lack of robust and reproducible changes in sgRNA abundance in response to CDC7i is also observed when MED12 and MED13 are targeted, which are required to facilitate the interaction between CDK8-CCNC and the transcriptional mediator complex (Dannappel et al., 2019; Fant and Taatjes, 2019; Menzl et al., 2019).

Taken together these observations suggest that loss of function of CDK8, and not its closely related paralog CDK19, sensitises cells to CDC7 inhibition, and that a role for CDK8 in supporting proliferation of cells with limited CDC7 kinase activity may be independent from its interaction and transcriptional role with the mediator complex.

### CDK8/CDK19 determine the potency of CDC7 inhibitors in proliferation and DNA replication assays

Recently, CDK8/CDK19-CCNC complex was shown to bind and phosphorylate the human replication initiation factor, MTBP, *in vitro* (Ferreira et al., 2021; Köhler et al., 2019). While the region of MTBP, required for binding CDK8/CDK19-CCNC complex, is important for normal genome replication, the requirement and role of CDK8/CDK19-CCNC in this process is poorly understood (Ferreira et al., 2021; Köhler et al., 2019). For this reason, we focused our analysis on determining their role in the response to CDC7 inhibition.

In order to independently demonstrate that CDK8 activity allows cells to grow better when CDC7 is inhibited, we performed a long-term proliferation assays (Figure 2a and b). Briefly, MCF10A cells were mock-treated or treated in the continued presence of a dual-specificity CDK8/CDK19 inhibitor, BI-1347, in the presence or absence of XL413 at two different concentrations (1.25µM and 10 µM) for a 7-day period. Relative cell proliferation was estimated by staining cells with crystal violet and measuring staining intensities. Compared to controls, the inhibition of CDK8/CDK19 with 31 nM BI-1347 alone resulted in a decrease in the relative staining intensity of approximately 30 % (Figure 2a and b). Inhibition of CDC7, with either 1.25 µM or 10 µM of XL413 alone resulted in a relative intensity reduction of ~15 % and 70 % respectively. Importantly the addition of BI-1347 to XL413 caused a further reduction in cell number with staining intensity decreasing by 70 % and 95 % respectively. These results strengthen the idea that inhibition of CDK8 activity confers a growth disadvantage under conditions of limited CDC7 activity.

We next sought to understand if the effects of CDK8 and CDC7 inhibition on proliferation were related to altered dynamics of the DNA replication. For these experiments MCF10A cells were either mock-treated or treated with XL413 for 24 hours in the presence or absence of BI-1347 at increasing concentrations. Flow cytometry was used to determine the consequences on cell cycle distribution and the rate of DNA synthesis (Figure 2c-f). As previously shown XL413 treatment strongly reduced the rate of DNA synthesis (Alver et al., 2017; Hiraga et al., 2017; Rainey et al., 2017, 2020), leading to a characteristic accumulation of cells in mid to late S phase (Figure 2c-e). Instead CDK8/CDK19 inhibition resulted in a dose-dependent increase in G1 phase cells with a corresponding decrease in early-mid S phase cells. Utilising a more refined analysis however we noticed a subtle and dose-independent decrease in the rate of DNA synthesis in S phase cells (Figure 2f). Strikingly, in XL413 treated cells inhibition of CDK8/CDK19 resulted in a further accumulation of mid-late S phase cells with a profound and dose independent decrease in the rate of DNA synthesis (Figure 2c-f). Together these results indicate CDK8/CDK19 activity is required to promote efficient DNA synthesis and such role in S-phase becomes critical when CDC7 activity becomes rate limiting.

By adopting the same experimental approach, we aimed to understand whether CDK8/CDK19 inhibition could sensitise cells CDC7 inhibitors across a wide range of concentrations. For these experiments and in order to rule out possible off-target effects we used two distinct CDC7 inhibitors, XL413 and TAK- 931 (Iwai et al., 2019). When cells were treated with either CDC7 inhibitor, in the absence of CDK8/CDK19i, we observed a dose-dependent accumulation of mid-late S phase cells and corresponding decrease in the rate of DNA synthesis of S phase cells (Figure 3a-f). Inhibition of CDK8/CDK19 alone had minimal impact on the cell cycle distributions and resulted in a slight decrease in rate of DNA synthesis of S phase cells. Strikingly, when together with the CDK8/CDK19 inhibitor both CDC7 inhibitors cased a more pronounced accumulation of cells in mid-late S phase of the cell cycle and much more profound decrease in rate of DNA synthesis at all doses tested.

Finally, to understand whether the decrease of DNA synthesis by CDK18/19 and CDC7 inhibitors is a general mechanism or is related to a nuance of MCF10A cells similar experiments were performed in a panel of cancer cell lines (MDA-MB-468, MDA-MB-231 and U2-O-S). In all these cell lines we observed a strong potentiation of cell cycle and DNA replication phenotypes imposed by CDC7 inhibition by the CDK8/CDK19 inhibitor BI-1347 (Supplemental Figure 3).

Taken together these results indicate that inhibition of CDK8/CDK19 can potently sensitise cells to the effects of CDC7 inhibition on DNA replication dynamics, and that CDK8/CDK19 activity can modulate cellular responses to CDC7 inhibition in multiple cell types.

### DISCUSSION

Successful drug development requires the understanding of which patent population is most likely to benefit of any given new drug as well as whether a new drug can be administered as single agents or in a combination therapy. Such understanding for CDC7 inhibitors is still very limited and mostly based on empirical testing in preclinical models.

We have embarked in finding the genes that can alter the response to CDC7 inhibitors using functional genomics approaches (Rainey et al., 2020). The genome wide-CRISPR/Cas9 screen presented in this work now indicates that loss of function of CDK8 and CCNC sensitizes cells to the anti-proliferative activity of CDC7 inhibition. This suggests that tumors defective in either CDK8 and/or CCNC could be particularly sensitive to CDC7i-basesd chemotherapy and that CDK8 and CCNC could be then developed as novel biomarkers of response. In a preliminary analysis of publicly available data form 10,967 cancer samples, form the cBioPortal TCGA PanCancer Atlas of 32 studies, we noted that CDK8 deletion is present in sarcomas (~4.31 % of 255 cases), lung adenocarcinomas (~2.47 % of 566 cases), and diffuse large B-cell lymphoma (~2.08 % of 48 cases). CCNC deletions instead is observed at high rate in diffuse large B-cell lymphoma (~12.5 % of 48 cases) prostate adenocarcinoma (~9.11 % of 494 cases) uveal melanoma (~7.5 % of 80 cases), liver hepatocellular carcinoma (~3.23 % of 372 cases) and stomach adenocarcinoma (~2.95 % of 440 cases), that show frequent deep of CCNC.

We have then shown that inhibition of CDK8/CDK19 kinases by BI-1347 potently sensitizes cells to the anti-proliferative effects of CDC7 inhibition. Mechanistically we have shown that, while per se CDK8/CDK19 have a minor contribution to the rate of DNA synthesis, their inhibition in combination with even the lowest dose of CDC7 inhibitors tested, elicits a profound decrease in the rate of DNA synthesis, that in our replication assays appears to be equivalent to a ~10-fold increase in the potency of the CDC7 inhibitor.

It is very likely that the synergistic decrease in proliferation and rate of DNA synthesis, observed upon combined CDK8/CDK19i and CDC7i, are not related to the role of CDK8 in transcription, but possibly to a direct role of CDK8 in DNA replication. In favour of this hypothesis is the fact that, while CDK8 was the top hit, neither MED12 nor MED13, which target CDK8 to the mediator complex for regulation transcription, nor the CDK19 paralogue, scored in our CRISPR screen.

Such more fundamental role of CDK8 in DNA replication also strongly suggests that a CDC7i + CDK8i combination will be effective in blocking cell proliferation in a very large variety of cancers, thus very much expanding the scope of CDK8 inhibition which at present is limited to a small subset of cancers that have strong dependencies on CDK8 mediated gene expression.

Finally we note that a recent study reported that siRNA mediated down regulation of CDK19 but not of CDK8 can prevent apoptosis induced by a dual specificity CDC7/CDK9 inhibitor, PHA767491, in several cancer cell lines (Sasi et al., 2017). Apoptosis induced by PHA-767491 maybe be driven by loss of anti-apoptotic proteins such as MCL1 (Natoni et al., 2011, 2013), among other gene expression changes, as a result of inhibition of CDK9. It plausible that CDK19 contributes to this apoptotic transcriptional response by regulating the expression of additional apoptotic genes. Such results are thus only apparently different from ours which instead indicate that only CDK8 is relevant to enhance proliferation when CDC7 is inhibited by XL413 and TAK931, which do not affect CDK9 activity.

In summary, we identify CDK8 and CCNC are potential novel biomarkers whose functional status can modulate the anti-cancer effects of CDC7i monotherapy. Furthermore we show, for the first time, that inhibition of CDK8/CDK19 potentiates the anti-proliferative activity a of CDC7 inhibition suggesting that a combination of CDC7 with CDK8 inhibitors may improve efficacy and can be potentially efficacious across a broader spectrum of cancers when compared to either monotherapy.

### METHODS

### EXPERIMENTAL MODEL AND SUBJECT DETAILS

### Cell culture

Cell culture was performed in a Class II Bio-safety cabinet and all cell lines were maintained at 37 °C in a humidified atmosphere containing 5% CO₂. Cell count and viability were determined using a LunaII automated cell counter (Logos Biosystems) and trypan blue exclusion. Culture media and reagents were from Sigma-Aldrich unless stated otherwise. MCF10A cell line (human, female origin) was purchased from ATCC and used without further authentication. MCF10A-EditR cells (stably expressing Cas9) were generated as previously described (Rainey et al., 2017). MCF10A cells and derivatives were cultured using DMEM supplemented with 5% horse serum, 25 ng/ml cholera toxin, 10 µg/ml insulin, 20 ng/ml epidermal growth factor (Peprotech), 500 ng/ml hydrocortisone, 50 U/ml penicillin and 50 µg/ml streptomycin. Lenti-X^{™} 293T cell line (human, female origin) were purchased from TaKaRa and used without further authentication. Routine culture was performed using DMEM +GlutMAX^{™}-I (ThermoFisher Scientific) supplemented with 10% fetal bovine serum, 50 U/ml penicillin and 50 µg/ml streptomycin. MDA-MB-468 (human, female origin) were cultured in RPMI-1640 medium (Sigma-Aldrich) supplemented with 2 mM L-glutamine, 10% fetal bovine serum, 50 U/ml penicillin and 50 µg/ml streptomycin. U-2 OS (human, female origin) and MDA-MB-231 (human, female origin) were cultured in DMEM (Sigma-Aldrich) supplemented with 10% fetal bovine serum, 2 mM L-Glutamine, 50 U/ml penicillin and 50 µg/ml streptomycin.

### METHOD DETAILS

### CRISPR Pooled Lentiviral sgRNA Library

A plasmid based CRISPR pooled lentiviral sgRNA library that can be used in a modular format was purchased from CELLECTA (CELLECTA: CRISPR Human Genome Knockout Library, Module 1 (200 µg plasmid), #sgRNA: 52,549, KOHGW-M1-P; Module 2 (200 µg plasmid), #sgRNA: 49,461, KOHGW-M2-P; and Module 3 (200 µg plasmid), #sgRNA: 48,578, KOHGW-M3-P). Each module covers approximately 6,300 genes and each gene is targeted by 8x sgRNAs (~50,000 sgRNAs per module), in total targeting >19,000 protein coding genes. Lentiviral production, functional titer estimation and pooled genome-wide CRISPR/Cas9 screening were performed according to user manual (CRISPR pooled lentiviral sgRNA libraries; www.cellecta.com) with minor modifications as outlined below.

### Library production

For lentivirus production, 25x10⁶ Lenti-X 293T cells were seeded on 20 X 150 mm plates for each sgRNA library module and transfected the following day. For each module, a master stock of packaging plasmid (600 µg) and sgRNA plasmid (120 µg) were combined in 20 ml of 150 mM NaCl and mixed with 1440 µl of polyethylenimine (1 mg/ml) that had been diluted in 20 ml of 150 mM NaCl. After 20 minutes of incubation 2 ml of the mix were added dropwise to each plate. 24 hours post-transfection, media was exchanged for DMEM medium supplemented with 10% FBS, DNase I (1 U/ml), 5 mM MgCl₂, 20 mM HEPES pH7.4. Lentiviruses were harvested 48 hours post-transduction and filtered through a Nalgene 0.2 µm PES filter. Lentifuge (1 µl per ml) was added to the filtrate and incubated at 4 °C for 1 hour. The filtrate was then centrifuged for 2 hours at 12,000 rpm and the pellet resuspended in 1/100 of the original volume of D-PBS (Sigma), aliquoted and frozen at -80 °C.

### Lentiviral titer determination

Aliquots of viral supernatant were titered by adding incremental volumes (0.1 µl to 1 µl for concentrated virus or 10 µl to 100 µl for viral supernatant) in the presence of polybrene (5 µg/ml) to MCF10A EditR cells that had been seeded 24 hours in advance at 120,000 cells per well in a 6 well plate. Media was changed after 24 hours and at 48 hours after transduction, MCF10A EditR cells were harvested and fixed in 4% paraformaldehyde in PBS for 20 minutes at room temperature before being washed and resuspended in PBS. Number of RFP (Cellecta sgRNA library) expressing cells was determined on a BD LSR FORTESSA 2 flow cytometer and analysis using FlowJo software. The number of transduction units per ml was determined using a titer chart (CELLECTA user manual: CRISPR pooled lentiviral sgRNA libraries; www.cellecta.com) with the following equation: TU per ml = (# of cells at Transduction) × [MOI / (ml of lentiviral stock used at transduction)].

### Pooled Genome-wide CRISPR/Cas9 screen and data analysis

MCF10A EditR cells (120 x 10⁶ per module) were transduced with individual modules (Modules I, II and III) in the presence of 5 µg/ml polybrene at a Multiplicity of Infection (MoI) of 0.4 achieving ~600-fold guide representation. After 24 hours, transduced cells were harvested, replated and selected with puromycin (1 µg/ml) for 6 days. Puromycin resistant cells were harvested and a sample of ~30 x10⁶ cells taken for analysis by next generation sequencing (NGS) at the start of the screen (day 0). The remaining puromycin resistant cells from each module were cultured independently from each other in untreated and XL413 treated (1.25 µM) experimental branches. Throughout the screen cell proliferation and viability were determined by cell counting on a LunaII automated cell counter (Logos Biosystems) using trypan blue exclusion. Cells were cultured, harvested, reseeded and retreated every 4 days in order to maintain ~600-fold guide representation. Finally at day 20 for the untreated branch and at day 24 for the XL413-treated branche, cells were harvested and a sample of ~30 x10⁶ cells taken for analysis by NGS.

### Genomic DNA extraction

Cell pellets were resuspended in 6 ml of Buffer P1 (50 mM Tris-Cl, pH 8.0, 10 mM EDTA, Qiagen) and lysed by addition of SDS to final concentration of 1 % (v/v). Samples were sonicated at 10 % amplitude for 5 seconds (Branson, digital sonifier) before addition of proteinase K (40 µg/ml, Qiagen) followed by gentle mixing and incubation at 55°C overnight. Samples were cooled to 37°C, and following addition of RNaseA (100 µg/ml, Qiagen), incubated for 30min. Samples were chilled on ice before addition of 2 ml of ice cold 7.5 M ammonium acetate (Sigma) followed by two repeat 20 second vortex-pulses and centrifugation at 4,000 g for 10 minutes. The supernatant was transferred to a 15 ml conical tube and genomic DNA was precipitated by addition 6 ml of Isopropanol followed by mixing and centrifugation at 4,000 g for 40 minutes. Genomic DNA was washed with 6 ml of 70 % (v/v) Ethanol, centrifuged at 4,000 g for 10 minutes and the genomic DNA pellet air-dried before being resuspended in 220ul of DNase/RNase free water and quantitated on a Nanodrop.

### sgRNA library amplification

Genomic DNA was subject to a first round PCR using multiple reactions, with a maximum of 50 µg of genomic DNA in each 100 µl PCR reaction, with 0.2 mM of each dNTP, 0.3 µM of each primer (Forward 5'-AGGGCCTATTTCCCATGATTCCT-3' (SEQ ID NO: 63) and Reverse 5'-CGACAACAACGCAGAAATTTTGAAT-3' (SEQ ID NO: 64)), and 1x Titanium *Taq* DNA polymerase in 1x Titanium *Taq* buffer. Genomic DNA was initially denatured at 94°C for 4 minutes, followed by 18 cycles of 94°C for 45 seconds, 63°C for 15 seconds, 72°C for 35 seconds, 1 cycle of 72°C for 4 minutes and hold at 4°C. All 1^{st} round PCR reactions for a sample were pooled, mixed and a 5ml aliquot used directly for 2^{nd} round PCR in a 100 µl final volume, as described before, except using 0.5 µM of each primer designed to add Illumina: flow-cell adaptors; barcodes and annealing sites for sequencing primers (NGS-Fwd1 5'-AATGATACGGCGACCACCGAGATCTACACTATAGCCTACACTCTTTCCCTACA CGACGCTCTTCCGATCTTTGTGGAAAGGACGAAACACCG-3' (SEQ ID NO: 65) and NGS-Rev2 5'-CAAGCAGAAGACGGCATACGAGATCGAGTAATGTGACTGGAGTTCAGACGTG TGCTCTTCCGATCTGAGGTTCAGAGTTCTACAGTCC-3' (SEQ ID NO: 66)). The 2^{nd} round PCR was subject to PCR clean-up (Macherey-Nagel), to concentrate the DNA into a 50 µl volume, followed by gel purification (Macherey-Nagel) of a DNA product of ~293bp from a 2 % (w/v) agarose DNA gel. Gel purified libraries were sent to Novagene for ilummina NGS analysis using a NovaSeq 6000 PE150, paired-end reads and 30Gb of raw data per sample. The libraries were shipped to Novagene for ilummina NGS analysis using a NovaSeq 6000 PE150, with paired-end reads and 30 Gb raw data per sample.

### NGS data analysis

FASTQ files obtained by NGS were analysed using the MAGeCKFlute pipeline (Wang et al., 2019) as outlined below. MAGeCK was used to perform adaptor trimming, mapping of trimmed sgRNAs and associated sequencing reads to the CELLECTA sgRNA reference library, and a quality control assessment of the sequencing quality and screen data. Following quality control inspection, sgRNA read count data from the day 0 vs day 20 untreated and day 0 vs day 24 XL413-treated (1.25 µM) conditions were modelled using a negative-binomial distribution by MAGeCK-MLE to calculate a beta score for each targeted gene. Negative control sgRNAs in the CELLECTA library were used by MAGeCK-MLE to normalize the read counts between samples. Beta score normalization was performed by MAGeCK-Flute, using the proposed list of core essential genes (Wang et al., 2019), to accommodate the effect of the CDC7 inhibitor on decreased cell proliferation throughout the screen. No copy number correction was applied as the MCF10A EditR cell line used in the screen are near diploid. The normalized control beta score was subtracted from normalized treatment beta score, for each gene target, and the genes were ranked and plotted based on this differential beta score. Gene hits were identified as outliers +/- 2x standard deviation of the mean differential beta score. For the hits identified, the normalised sgRNA count data was plotted and used to compare the relative abundance in day 20 untreated and day 24 XL413-treated (1.25 µM) samples to day 0.

### Cell proliferation assay

MCF10A cells were seeded at 4,000 cells per well of a 6 well plate and after 24 hours were treated with inhibitors before being incubated for 7 days at 37 °C with 5% CO₂. Media was removed from the cells which were then washed with PBS and air dried before being fixed with 100 % methanol for 1 hour. Following removal of methanol the plates were air-dried, stained with crystal violet staining solution (0.2% crystal violet, 25% methanol) for 1 hour at room temperature, washed with ddH₂O and air-dried. Plates were scanned using a HP Scanjet 4050, to generate images of crystal violet stained cells, and crystal violet staining intensity was acquired using the Odyssey infrared imaging system and Image Studio software (LI-COR Biosciences).

### Flow cytometry

To analyse the cell cycle and DNA synthesis, nascent DNA was labelled by incubating cells with 10 µM EdU for either 20 or 30 minutes prior to harvest, washing with PBS, and then by resuspension in PBS containing 70% EtOH for 1 hour at -20 °C. After a PBS wash, incorporated EdU was labelled by incubating cells in click reaction buffer (PBS containing 10 µM 6-Carboxyfluorescine-TEG-azide, 10 mM Sodium-L-ascorbate, 2 mM Copper-II-Sulphate) for 30 minutes protected from light. Cells were washed in PBS containing 1% BSA, 5% Tween 20 and then DNA stained in PBS containing 1% BSA and 1 µg/ml DAPI. All washes involve: centrifugation at 400 x g for 5 minutes, removal of supernatant from pellet, resuspension in the appropriate buffer, recentrifugation and removal of supernatant from the pellet prior to the next step.

Fluorescence intensity data for 6-Carboxyfluorescine (488_530_30 nm) and DAPI (405_450_50 nm) were acquired for 10,000 single cells on a BD FACS Canto II and analysed using FlowJo software. To further analyse fluorescence intensity, proportional to EdU incorporation, for individual cells, gates were applied on DAPI-EdU biparametric dot plots to select for EdU positive S phase cells using FlowJo. According to the required analysis these gates included either all S phase cells, or only early or late S phase cells. Fluorescence intensity (488_530_30) values per cell were exported and plotted as scatter dot plots using GraphPad prism. For comparisons of mean fluorescence intensity of a cell population across three biological repeat experiments, all fluorescence intensity values were normalised to the maximum fluorescence intensity of the untreated control sample of each respective experiment to account for absolute differences in EdU staining. The mean fluorescence intensity values of these cell populations across three biological repeat experiments are expressed as a ratio relative to a control sample and plotted as a scatter dot plot using GraphPad prism.

### Quantification and Statistical analysis

GraphPad PRISM 8.4.2 for windows was used to generate all graphs and perform statistical test. Information about the statistical tests and the number of repeat experiments analysed are provided in the figure legends.

### REFERENCES

Adler, A.S., McCleland, M.L., Truong, T., Lau, S., Modrusan, Z., Soukup, T.M., Roose-Girma, M., Blackwood, E.M., and Firestein, R. (2012). CDK8 Maintains Tumor Dedifferentiation and Embryonic Stem Cell Pluripotency. Cancer Res. 72, 2129-2139. Akamatsu, M., Mikami, N., Ohkura, N., Kawakami, R., Kitagawa, Y., Sugimoto, A., Hirota, K., Nakamura, N., Ujihara, S., Kurosaki, T., et al. (2019). Conversion of antigen-specific effector/memory T cells into Foxp3-expressing Tregcells by inhibition of CDK8/19. Sci. Immunol. 4.
Alver, R.C., Chadha, G.S., Gillespie, P.J., and Blow, J.J. (2017). Reversal of DDK-Mediated MCM Phosphorylation by Rif1-PP1 Regulates Replication Initiation and Replisome Stability Independently of ATR/Chk1. Cell Rep. 18, 2508.
Arnett, A., Moo, K.G., Flynn, K.J., Sundberg, T.B., Johannessen, L., Shamji, A.F., Gray, N.S., Decker, T., Zheng, Y., Gersuk, V.H., et al. (2021). The Cyclin-Dependent Kinase 8 (CDK8) Inhibitor DCA Promotes a Tolerogenic Chemical Immunophenotype in CD4 + T Cells via a Novel CDK8-GATA3-FOXP3 Pathway . Mol. Cell. Biol. 41.
Berge, S.M., Bighley, L.D., and Monkhouse, D.C. (1977). Pharmaceutical salts. J. Pharm. Sci. 66, 1-19.
Boos, D., Yekezare, M., and Diffley, J.F.X. (2013). Identification of a heteromeric complex that promotes DNA replication origin firing in human cells. Science (80-. ). 340, 981-984.
Chen, M., Li, J., Liang, J., Thompson, Z.S., Kathrein, K., Broude, E. V., and Roninson, I.B. (2019). Systemic Toxicity Reported for CDK8/19 Inhibitors CCT251921 and MSC2530818 Is Not Due to Target Inhibition. Cells 8.
Cheng, A.N., Lo, Y.K., Lin, Y.S., Tang, T.K., Hsu, C.H., Hsu, J.T.A., and Lee, A.Y.L. (2018). Identification of Novel Cdc7 Kinase Inhibitors as Anti-Cancer Agents that Target the Interaction with Dbf4 by the Fragment Complementation and Drug Repositioning Approach. EBioMedicine 36, 241.
Clarke, P.A., Ortiz-Ruiz, M.-J., TePoele, R., Adeniji-Popoola, O., Box, G., Court, W., Czasch, S., El Bawab, S., Esdar, C., Ewan, K., et al. (2016). Assessing the mechanism and therapeutic potential of modulators of the human Mediator complex-associated protein kinases. Elife 5.
Cowley, G.S., Weir, B.A., Vazquez, F., Tamayo, P., Scott, J.A., Rusin, S., East-Seletsky, A., Ali, L.D., Gerath, W.F.J., Pantel, S.E., et al. (2014). Parallel genome-scale loss of function screens in 216 cancer cell lines for the identification of context-specific genetic dependencies. Sci. Data 1, 1-12.
Czodrowski, P., Mallinger, A., Wienke, D., Esdar, C., Pöschke, O., Busch, M., Rohdich, F., Eccles, S.A., Ortiz-Ruiz, M.J., Schneider, R., et al. (2016). Structure-Based Optimization of Potent, Selective, and Orally Bioavailable CDK8 Inhibitors Discovered by High-Throughput Screening. J. Med. Chem. 59, 9337-9349.
Dannappel, M.V., Sooraj, D., Loh, J.J., Firestein, R., MV, D., D, S., JJ, L., and R, F. (2019). Molecular and in vivo Functions of the CDK8 and CDK19 Kinase Modules. Front. Cell Dev. Biol. 6, 171.
Deady, L.W. (1977). Ring Nitrogen Oxidation of Amino Substituted Nitrogen Heterocycles with M-Chloroperbenzoic Acid. Synth. Commun. 7, 509-514.
Ermoli, A., Bargiotti, A., Brasca, M.G., Ciavolella, A., Colombo, N., Fachin, G., Isacchi, A., Menichincheri, M., Molinari, A., Montagnoli, A., et al. (2009). Cell division cycle 7 kinase inhibitors: 1H-pyrrolo[2,3-b]pyridines, synthesis and structure-activity relationships. J. Med. Chem. 52, 4380-4390.
Ettl, T., Schulz, D., and Bauer, R.J. (2022). The Renaissance of Cyclin Dependent Kinase Inhibitors. Cancers (Basel). 14.
Fant, C.B., and Taatjes, D.J. (2019). Regulatory functions of the Mediator kinases CDK8 and CDK19. Transcription 10, 76-90.
Ferreira, P., Höfer, V., Kronshage, N., Marko, A., Reusswig, K.U., Tetik, B., Dießel, C., Köhler, K., Tschernoster, N., Altmüller, J., et al. (2021). MTBP phosphorylation controls DNA replication origin firing. Sci. Rep. 11.
Firestein, R., Bass, A.J., Kim, S.Y., Dunn, I.F., Silver, S.J., Guney, I., Freed, E., Ligon, A.H., Vena, N., Ogino, S., et al. (2008). CDK8 is a colorectal cancer oncogene that regulates β-catenin activity. Nature 455, 547-551.
Firestein, R., Shima, K., Nosho, K., Irahara, N., Baba, Y., Bojarski, E., Giovannucci, E.L., Hahn, W.C., Fuchs, C.S., and Ogino, S. (2010). CDK8 expression in 470 colorectal cancers in relation to beta-catenin activation, other molecular alterations and patient survival. Int. J. Cancer 126, 2863-2873.
Galbraith, M.D., Donner, A.J., and Espinosa, J.M. (2010). CDK8: A positive regulator of transcription. Transcription 1, 4-12.
Gilbert, D.M., Takebayashi, S.-I., Ryba, T., Lu, J., Pope, B.D., Wilson, K.A., and Hiratani, I. (2010). Space and Time in the Nucleus: Developmental Control of Replication Timing and Chromosome Architecture. Cold Spring Harb. Symp. Quant. Biol. 75, 143-153.
Guo, Y., Wang, J., Benedict, B., Yang, C., van Gemert, F., Ma, X., Gao, D., Wang, H., Zhang, S., Lieftink, C., et al. (2021). Targeting CDC7 potentiates ATR-CHK1 signaling inhibition through induction of DNA replication stress in liver cancer. Genome Med. 13*.*
Harper, T.M., and Taatjes, D.J. (2018). The complex structure and function of Mediator. J. Biol. Chem. 293, 13778-13785.
Hatcher, J.M., Wang, E.S., Johannessen, L., Kwiatkowski, N., Sim, T., and Gray, N.S. (2018). Development of Highly Potent and Selective Steroidal Inhibitors and Degraders of CDK8. ACS Med. Chem. Lett. 9, 540-545.
Hatcher, J.M., Vatsan, P.S., Wang, E., Jiang, J., and Gray, N.S. (2021). Development of Highly Potent and Selective Pyrazolopyridine Inhibitor of CDK8/19. ACS Med. Chem. Lett. 12, 1689-1693.
Hiraga, S.-I., Ly, T., Garzón, J., Hořejší, Z., Ohkubo, Y.-N., Endo, A., Obuse, C., Boulton, S.J., Lamond, A.I., and Donaldson, A.D. (2017). Human RIF1 and protein phosphatase 1 stimulate DNA replication origin licensing but suppress origin activation. EMBO Rep. 18, 403-419.
Hofmann, M.H., Mani, R., Engelhardt, H., Impagnatiello, M.A., Carotta, S., Kerenyi, M., Lorenzo-Herrero, S., Böttcher, J., Scharn, D., Arnhof, H., et al. (2020). Selective and potent CDK8/19 inhibitors enhance NK-cell activity and promote tumor surveillance. Mol. Cancer Ther. 19, 1018-1030.
Irie, T., Asami, T., Sawa, A., Uno, Y., Hanada, M., Taniyama, C., Funakoshi, Y., Masai, H., and Sawa, M. (2017). Discovery of novel furanone derivatives as potent Cdc7 kinase inhibitors. Eur. J. Med. Chem. 130, 406-418.
Irie, T., Asami, T., Sawa, A., Uno, Y., Taniyama, C., Funakoshi, Y., Masai, H., and Sawa, M. (2021). Discovery of AS-0141, a Potent and Selective Inhibitor of CDC7 Kinase for the Treatment of Solid Cancers. J. Med. Chem. 64, 14153-14164.
Iwai, K., Nambu, T., Dairiki, R., Ohori, M., Yu, J., Burke, K., Gotou, M., Yamamoto, Y., Ebara, S., Shibata, S., et al. (2019). Molecular mechanism and potential target indication of TAK-931, a novel CDC7-selective inhibitor. Sci. Adv. 5*.*
Iwai, K., Nambu, T., Kashima, Y., Yu, J., Eng, K., Miyamoto, K., Kakoi, K., Gotou, M., Takeuchi, T., Kogame, A., et al. (2021). A CDC7 inhibitor sensitizes DNA-damaging chemotherapies by suppressing homologous recombination repair to delay DNA damage recovery. Sci. Adv. 7, 197-218.
Johannessen, L., Sundberg, T.B., O'Connell, D.J., Kolde, R., Berstler, J., Billings, K.J., Khor, B., Seashore-Ludlow, B., Fassl, A., Russell, C.N., et al. (2017). Small-molecule studies identify CDK8 as a regulator of IL-10 in myeloid cells. Nat. Chem. Biol. 13, 1102-1108.
Köhler, K., Sanchez-Pulido, L., Höfer, V., Marko, A., Ponting, C.P., Snijders, A.P., Feederle, R., Schepers, A., and Boos, D. (2019). The Cdk8/19-cyclin C transcription regulator functions in genome replication through metazoan Sld7.
Koltun, E.S., Tsuhako, A.L., Brown, D.S., Aay, N., Arcalas, A., Chan, V., Du, H., Engst, S., Ferguson, K., Franzini, M., et al. (2012). Discovery of XL413, a potent and selective CDC7 inhibitor. Bioorg. Med. Chem. Lett. 22, 3727-3731.
Kurasawa, O., Homma, M., Oguro, Y., Miyazaki, T., Mori, K., Uchiyama, N., Iwai, K., Ohashi, A., Hara, H., Yoshida, S., et al. (2017). 2-Aminomethylthieno[3,2-d]pyrimidin-4(3H)-ones bearing 3-methylpyrazole hinge binding moiety: Highly potent, selective, and time-dependent inhibitors of Cdc7 kinase. Bioorg. Med. Chem. 25, 3658-3670.
Kurasawa, O., Miyazaki, T., Homma, M., Oguro, Y., Imada, T., Uchiyama, N., Iwai, K., Yamamoto, Y., Ohori, M., Hara, H., et al. (2020). Discovery of a Novel, Highly Potent, and Selective Thieno[3,2- d]pyrimidinone-Based Cdc7 Inhibitor with a Quinuclidine Moiety (TAK-931) as an Orally Active Investigational Antitumor Agent. J. Med. Chem. 63, 1084-1104.
Labib, K. (2010). How do Cdc7 and cyclin-dependent kinases trigger the initiation of chromosome replication in eukaryotic cells? Genes Dev. 24, 1208-1219.
Liang, J., Chen, M., Hughes, D., Chumanevich, A.A., Altilia, S., Kaza, V., Lim, C.U., Kiaris, H., Mythreye, K., Pena, M.M., et al. (2018). CDK8 Selectively Promotes the Growth of Colon Cancer Metastases in the Liver by Regulating Gene Expression of TIMP3 and Matrix Metalloproteinases. Cancer Res. 78, 6594-6606.
Lloyd, R.L., Urban, V., Muñoz-Martínez, F., Ayestaran, I., Thomas, J.C., De Renty, C., O'Connor, M.J., Forment, J. V., Galanty, Y., and Jackson, S.P. (2021). Loss of Cyclin C or CDK8 provides ATR inhibitor resistance by suppressing transcription-associated replication stress. Nucleic Acids Res. 49*.*
Mallinger, A., Crumpler, S., Pichowicz, M., Waalboer, D., Stubbs, M., Adeniji-Popoola, O., Wood, B., Smith, E., Thai, C., Henley, A.T., et al. (2015). Discovery of potent, orally bioavailable, small-molecule inhibitors of WNT signaling from a cell-based pathway screen. J. Med. Chem. 58, 1717-1735.
Mallinger, A., Schiemann, K., Rink, C., Stieber, F., Calderini, M., Crumpler, S., Stubbs, M., Adeniji-Popoola, O., Poeschke, O., Busch, M., et al. (2016). Discovery of Potent, Selective, and Orally Bioavailable Small-Molecule Modulators of the Mediator Complex-Associated Kinases CDK8 and CDK19. J. Med. Chem. 59, 1078-1101.
Masai, H., Taniyama, C., Ogino, K., Matsui, E., Kakusho, N., Matsumoto, S., Kim, J.-M., Ishii, A., Tanaka, T., Kobayashi, T., et al. (2006). Phosphorylation of MCM4 by Cdc7 kinase facilitates its interaction with Cdc45 on the chromatin. J. Biol. Chem. 281, 39249-39261.
McDermott, M.S.J., Chumanevich, A.A., Lim, C.U., Liang, J., Chen, M., Altilia, S., Oliver, D., Rae, J.M., Shtutman, M., Kiaris, H., et al. (2017). Inhibition of CDK8 mediator kinase suppresses estrogen dependent transcription and the growth of estrogen receptor positive breast cancer. Oncotarget 8, 12558-12575.
McDonald, E.R., de Weck, A., Schlabach, M.R., Billy, E., Mavrakis, K.J., Hoffman, G.R., Belur, D., Castelletti, D., Frias, E., Gampa, K., et al. (2017). Project DRIVE: A Compendium of Cancer Dependencies and Synthetic Lethal Relationships Uncovered by Large-Scale, Deep RNAi Screening. Cell 170, 577-592.e10.
McFarland, J.M., Ho, Z. V., Kugener, G., Dempster, J.M., Montgomery, P.G., Bryan, J.G., Krill-Burger, J.M., Green, T.M., Vazquez, F., Boehm, J.S., et al. (2018). Improved estimation of cancer dependencies from large-scale RNAi screens using model-based normalization and data integration. Nat. Commun. 9, 4610.
Menichincheri, M., Bargiotti, A., Berthelsen, J., Bertrand, J.A., Bossi, R., Ciavolella, A., Cirla, A., Cristiani, C., Croci, V., D'Alessio, R., et al. (2009). First Cdc7 kinase inhibitors: pyrrolopyridinones as potent and orally active antitumor agents. 2. Lead discovery. J. Med. Chem. 52, 293-307.
Menichincheri, M., Albanese, C., Alli, C., Ballinari, D., Bargiotti, A., Caldarelli, M., Ciavolella, A., Cirla, A., Colombo, M., Colotta, F., et al. (2010). Cdc7 kinase inhibitors: 5-heteroaryl-3-carboxamido-2-aryl pyrroles as potential antitumor agents. 1. Lead finding. J. Med. Chem. 53, 7296-7315.
Menzl, I., Witalisz-Siepracka, A., and Sexl, V. (2019). CDK8-novel therapeutic opportunities. Pharmaceuticals 12.
Meyers, R.M., Bryan, J.G., McFarland, J.M., Weir, B.A., Sizemore, A.E., Xu, H., Dharia, N. V, Montgomery, P.G., Cowley, G.S., Pantel, S., et al. (2017). Computational correction of copy number effect improves specificity of CRISPR-Cas9 essentiality screens in cancer cells. Nat. Genet. 49, 1779-1784.
Montagnoli, A., Tenca, P., Sola, F., Carpani, D., Brotherton, D., Albanese, C., and Santocanale, C. (2004). Cdc7 Inhibition Reveals a p53-Dependent Replication Checkpoint That Is Defective in Cancer Cells. Cancer Res. 64, 7110-7116.
Montagnoli, A., Valsasina, B., Croci, V., Menichincheri, M., Rainoldi, S., Marchesi, V., Tibolla, M., Tenca, P., Brotherton, D., Albanese, C., et al. (2008). A Cdc7 kinase inhibitor restricts initiation of DNA replication and has antitumor activity. Nat. Chem. Biol. 4, 357-365.
Montagnoli, A., Moll, J., and Colotta, F. (2010). Targeting cell division cycle 7 kinase: A new approach for cancer therapy. Clin. Cancer Res. 16, 4503-4508.
Nakamura, A., Nakata, D., Kakoi, Y., Kunitomo, M., Murai, S., Ebara, S., Hata, A., and Hara, T. (2018). CDK8/19 inhibition induces premature G1/S transition and ATR-dependent cell death in prostate cancer cells. Oncotarget 9.
Natoni, A., Murillo, L.S., Kliszczak, A.E., Catherwood, M.A., Montagnoli, A., Samali, A., O'Dwyer, M., and Santocanale, C. (2011). Mechanisms of action of a dual Cdc7/Cdk9 kinase inhibitor against quiescent and proliferating CLL cells. Mol. Cancer Ther. 10, 1624-1634.
Natoni, A., Coyne, M.R.E., Jacobsen, A., Rainey, M.D., O'Brien, G., Healy, S., Montagnoli, A., Moll, J., O'Dwyer, M., and Santocanale, C. (2013). Characterization of a Dual CDC7/CDK9 Inhibitor in Multiple Myeloma Cellular Models. Cancers (Basel). 5, 901-918.
Nemet, J., Jelicic, B., Rubelj, I., and Sopta, M. (2014). The two faces of Cdk8, a positive/negative regulator of transcription. Biochimie 97, 22-27.
Pelish, H.E., Liau, B.B., Nitulescu, I.I., Tangpeerachaikul, A., Poss, Z.C., Da Silva, D.H., Caruso, B.T., Arefolov, A., Fadeyi, O., Christie, A.L., et al. (2015). Mediator kinase inhibition further activates super-enhancer-associated genes in AML. Nature 526, 273-276.
Philip, S., Kumarasiri, M., Teo, T., Yu, M., and Wang, S. (2018). Cyclin-Dependent Kinase 8: A New Hope in Targeted Cancer Therapy? J. Med. Chem. 61, 5073-5092.
Porter, D.C., Farmaki, E., Altilia, S., Schools, G.P., West, D.K., Chen, M., Chang, B.D., Puzyrev, A.T., Lim, C.U., Rokow-Kittell, R., et al. (2012). Cyclin-dependent kinase 8 mediates chemotherapy-induced tumor-promoting paracrine activities. Proc. Natl. Acad. Sci. U. S. A. 109, 13799-13804.
Poss, Z.C., Ebmeier, C.C., Odell, A.T., Tangpeerachaikul, A., Lee, T., Pelish, H.E., Shair, M.D., Dowell, R.D., Old, W.M., and Taatjes, D.J. (2016). Identification of Mediator kinase substrates in human cells using cortistatin A and quantitative phosphoproteomics. Cell Rep. 15, 436.
Putz, E.M., Gotthardt, D., Hoermann, G., Csiszar, A., Wirth, S., Berger, A., Straka, E., Rigler, D., Wallner, B., Jamieson, A.M., et al. (2013). CDK8-mediated STAT1-S727 phosphorylation restrains NK cell cytotoxicity and tumor surveillance. Cell Rep. 4, 437-444.
Rainey, M.D., Quachthithu, H., Gaboriau, D., and Santocanale, C. (2017). DNA Replication Dynamics and Cellular Responses to ATP Competitive CDC7 Kinase Inhibitors. ACS Chem. Biol. 12, 1893-1902.
Rainey, M.D., Bennett, D., O'Dea, R., Zanchetta, M.E., Voisin, M., Seoighe, C., and Santocanale, C. (2020). ATR Restrains DNA Synthesis and Mitotic Catastrophe in Response to CDC7 Inhibition. Cell Rep. 32*.*
Remington, J.P. (Joseph P., and Osol, A. (1980). Remington's Pharmaceutical sciences, (ISBN: 9780912374024). 1928.
Rzymski, T., Mikula, M., Zylkiewicz, E., Dreas, A., Wiklik, K., Golas, A., Wójcik, K., Masiejczyk, M., Wróbel, A., Dolata, I., et al. (2017). SEL120-34A is a novel CDK8 inhibitor active in AML cells with high levels of serine phosphorylation of STAT1 and STATS transactivation domains. Oncotarget 8, 33779-33795.
Sasi, N.K., Tiwari, K., Soon, F.F., Bonte, D., Wang, T., Melcher, K., Xu, H.E., and Weinreich, M. (2014). The potent Cdc7-Dbf4 (DDK) kinase inhibitor XL413 has limited activity in many cancer cell lines and discovery of potential new DDK inhibitor scaffolds. PLoS One 9.
Sasi, N.K., Bhutkar, A., Lanning, N.J., MacKeigan, J.P., and Weinreich, M. (2017). DDK Promotes Tumor Chemoresistance and Survival via Multiple Pathways. Neoplasia 19, 439.
Seo, J.O., Han, S.I., and Lim, S.C. (2010). Role of CDK8 and β-catenin in colorectal adenocarcinoma. Oncol. Rep. 24, 285-291.
Sheu, Y.-J., and Stillman, B. (2006). Cdc7-Dbf4 phosphorylates MCM proteins via a docking site-mediated mechanism to promote S phase progression. Mol. Cell 24, 101-113.
Sheu, Y.-J., and Stillman, B. (2010). The Dbf4-Cdc7 kinase promotes S phase by alleviating an inhibitory activity in Mcm4. Nature 463, 113-117.
Stahl, P.H., Wermuth, C.G., and International Union of Pure and Applied Chemistry. (2011). Handbook of pharmaceutical salts : properties, selection, and use. 446.
Teesdale-Spittle, P. (1993). Advanced organic chemistry: Reactions, mechanisms and structure, 4th edn, Jerry March, Wiley, New York, 1992, 1512 pp. £24.95 (paperback), £45.50 (cloth). ISBN 0471581488. Appl. Organomet. Chem. 7, 293-293.
Tsherniak, A., Vazquez, F., Montgomery, P.G., Weir, B.A., Kryukov, G., Cowley, G.S., Gill, S., Harrington, W.F., Pantel, S., Krill-Burger, J.M., et al. (2017). Defining a Cancer Dependency Map. Cell 170, 564-576.e16.
Tsutsui, T., Umemura, H., Tanaka, A., Mizuki, F., Hirose, Y., and Ohkuma, Y. (2008). Human mediator kinase subunit CDK11 plays a negative role in viral activator VP16-dependent transcriptional regulation. Genes to Cells 13, 817-826.
Vanotti, E., Amici, R., Bargiotti, A., Berthelsen, J., Bosotti, R., Ciavolella, A., Cirla, A., Cristiani, C., D'Alessio, R., Forte, B., et al. (2008). Cdc7 kinase inhibitors: pyrrolopyridinones as potential antitumor agents. 1. Synthesis and structure-activity relationships. J. Med. Chem. 51, 487-501.
Wang, B., Wang, M., Zhang, W., Xiao, T., Chen, C.-H., Wu, A., Wu, F., Traugh, N., Wang, X., Li, Z., et al. (2019). Integrative analysis of pooled CRISPR genetic screens using MAGeCKFlute. Nat. Protoc. 14, 756-780.
Wermuth, C.G., and Stahl, P.H. (2002). IUPAC Pharmaceutical Salts: Properties, Selection, and Use A Handbook. Chem. Int 24, 1-2.
Xi, M., Chen, T., Wu, C., Gao, X., Wu, Y., Luo, X., Du, K., Yu, L., Cai, T., Shen, R., et al. (2019). CDK8 as a therapeutic target for cancers and recent developments in discovery of CDK8 inhibitors. Eur. J. Med. Chem. 164, 77-91.
Zhang, L., Cheng, C., Li, J., Wang, L., Chumanevich, A.A., Porter, D.C., Mindich, A., Gorbunova, S., Roninson, I.B., Chen, M., et al. (2022). A Selective and Orally Bioavailable Quinoline-6-Carbonitrile-Based Inhibitor of CDK8/19 Mediator Kinase with Tumor-Enriched Pharmacokinetics. J. Med. Chem. 65, 3420-3433.

### SEQUENCES

The mRNA sequences below represent the longest transcripts and encode the longest isoforms for: CDC7 (NM_003503.4), CDK8 (NM_001260.3) and CCNC (NM_005190.4), respectively. In each case the coding sequences is highlighted by an underline.
**Homo sapiens cell division cycle 7 (CDC7), transcript variant 1, mRNA** (SEQ ID **NO: 1)**
   **NCBI Reference Sequence: NM_003503.4** 
1 gttgcgcagg cgcatccgat cgactcggta ggtggggatc tcttggagac ggcgacccag

61 gcatctgggg agccacagaa gtcgtactcc cttaaaccct gctttgctcc ccctgtggat

121 gtaacccctt agctggcatt ttgcatctca attggcttgt gatggaggcg tctttgggga

181 ttcagatgga tgagccaatg gctttttctc cccagcgtga ccggtttcag gctgaaggct

241 ctttaaaaaa aaacgagcag aattttaaac ttgcaggtgt taaaaaagat attgagaagc

301 tttatgaagc tgtaccacag cttagtaatg tgtttaagat tgaggacaaa attggagaag

361 gcactttcag ctctgtttat ttggccacag cacagttaca agtaggacct gaagagaaaa

421 ttgctctaaa acacttgatt ccaacaagtc atcctataag aattgcagct gaacttcagt

481 gcctaacagt ggctgggggg caagataatg tcatgggagt taaatactgc tttaggaaga

541 atgatcatgt agttattgct atgccatatc tggagcatga gtcgtttttg gacattctga

601 attctctttc ctttcaagaa gtacgggaat atatgcttaa tctgttcaaa gctttgaaac

661 gcattcatca gtttggtatt gttcaccgtg atgttaagcc cagcaatttt ttatataata
 
721 ggcgcctgaa aaagtatgcc ttggtagact ttggtttggc ccaaggaacc catgatacga

781 aaatagagct tcttaaattt gtccagtctg aagctcagca ggaaaggtgt tcacaaaaca

841 aatcccacat aatcacagga aacaagattc cactgagtgg cccagtacct aaggagctgg

901 atcagcagtc caccacaaaa gcttctgtta aaagacccta cacaaatgca caaattcaga

961 ttaaacaagg aaaagacgga aaggagggat ctgtaggcct ttctgtccag cgctctgttt

1021 ttggagaaag aaatttcaat atacacagct ccatttcaca tgagagccct gcagtgaaac

1081 tcatgaagca gtcaaagact gtggatgtac tgtctagaaa gttagcaaca aaaaagaagg

1141 ctatttctac aaaagttatg aatagtgctg tgatgaggaa aactgccagt tcttgcccag

1201 ctagcctgac ctgtgactgc tatgcaacag ataaagtttg tagtatttgc ctttcaaggc

1261 gtcagcaggt tgcccctagg gcaggtacac caggattcag agcaccagag gtcttgacaa

1321 agtgccccaa tcaaactaca gcaattgaca tgtggtctgc aggtgtcata tttctttctt

1381 tgcttagtgg acgatatcca ttttataaag caagtgatga tttaactgct ttggcccaaa

1441 ttatgacaat taggggatcc agagaaacta tccaagctgc taaaactttt gggaaatcaa

1501 tattatgtag caaagaagtt ccagcacaag acttgagaaa actctgtgag agactcaggg

1561 gtatggattc tagcactccc aagttaacaa gtgatataca agggcatgct tctcatcaac

1621 cagctatttc agagaagact gaccataaag cttcttgcct cgttcaaaca cctccaggac

1681 aatactcagg gaattcattt aaaaaggggg atagtaatag ctgtgagcat tgttttgatg

1741 agtataatac caatttagaa ggctggaatg aggtacctga tgaagcttat gacctgcttg

1801 ataaacttct agatctaaat ccagcttcaa gaataacagc agaagaagct ttgttgcatc

1861 catttttaa agatatgagc ttgtgataat ggatcttcat ttaatgttta ctgttatgag

1921 gtagaataaa aaagaatact ttgtaatagc cacaagttct tgtttagaga ccagagcagg

1981 attaataatt tattttaaca ttttagtgtt tggtggcaca ttctaaaata tagattaaga

2041 atacttaaaa tgcctgggat agttcttggg actaacaaca tgatcttctt tgagttaaac

2101 ctacctaagt agattttagg tgggttccta ttaggtcaga tttttagctt ccctaattac
 
2161 ctttcactga catatacaga aaaaggagca gttttagttt taattaatta aaattaacag

2221 atgtgatgag gattaaatga atcaaaagac ttaatttgta gattctttta gagttatgag

2281 ctaggtatag tttggggaaa ctcaacctgg tgctggtgct cttaacaatt ttgtaaataa

2341 agaagataat ttccttttct agaggtacat attaggcctt ttatgaacac taaaacaatg

2401 aggaaatgtt ggtcatgggg caaagtatca cttaaaattg aattcatcca tttttaaaaa

2461 acacttcatg aaagcattct ggtgtgaatt gccatttttt tcttactggc ttctcaattt

2521 tcttccttct ctgcccctac ctaaaacatt ctcctcggaa attacatggt gctgaccaca

2581 aagtttctgg atgttttatt aaatattgta cgtgtttaca gttgggaatt taaaataata

2641 catacactgg ttgataaagg gaagctgcag gaccaaggtg aagattgata gtccaaatgc

2701 ttttcttttt tgagttgtat attttttcac accatcttag atataattag gtagctgctg

2761 aaaggaaaag tgaatacaga attgacggta ttattggaga tttttcctct gcgtagagcc

2821 atccagatct ctgtatcctg ttttgactaa gtcttaggtg ggttgggaag acagataatg

2881 aagtaggcaa agagaaaagg acccaagata gaggtttata ttcagaaatg gtatatatca

2941 atgacagcat atcaaacttc ctatgggaaa aagtctggtg ggtggtcagc tgacagattt

3001 cccatttagt agtcatagaa tacagaaata gtttagggac atgtattcat tttgttattt

3061 tgagcattga taggtcagta tatctaccta atctgtttgg taagtatagg atatataaac

3121 cattaccatt gatctgtctt atgccataat cttaaaaaaa atttgaatgc tcttgaattt

3181 gtatattcaa taaagttatc cttttatatt tttta
**Homo sapiens cyclin dependent kinase 8 (CDK8), transcript variant 1, mRNA (SEQ ID NO: 2)**
   **NCBI reference sequence: NM_001260.3:** 
1 gaggaggtac cggctgttgt gcggctctgc ccttctgttt gagtgtatgg gagagtgagt

61 gagtgagtga gtgtgagcgt gtgtgtgaga gcgtgaggcg tgagtgcgcg tgtgagagga

121 cgagagcccg cctggccgcc ccgccgctcc cgccgcagca ggagcagaac gcgcggccgg

181 agagagcggc ggagccggcg cccagggagc ccgcggggac aagggcagag acaccgctcc

241 ccacccccag ccctcgtccc tcggctctcc ttcgccgggg gatcctcccc gttcctccac

301 ccccggccgg cctctgcccc gccgtccccc tggatgtccc tggcgctttc gcggggcctc

361 ctcctgctct tgccgcatca gtcgggctgg tgctgcggcc ggcgggcgta gagcgggcgg

421 gttcccgggg gctgcggctg cccgtgcttc cccggtcccc acccctgccc cccggccccc

481 cgacccagct ctccggcctc agaggctgtg acaatggact atgactttaa agtgaagctg

541 agcagcgagc gggagcgggt cgaggacctg tttgaatacg agggctgcaa agttggccga

601 ggcacttatg gtcacgtcta caaagccaag aggaaagatg ggaaggatga taaagactat

661 gctttaaaac aaatagaagg aactgggatc tctatgtcgg catgtagaga aatagcatta

721 cttcgagagc ttaagcatcc aaacgtcatt tctcttcaaa aggtgtttct gtctcatgct

781 gataggaagg tgtggcttct gtttgactat gctgaacatg acctctggca tataatcaag

841 tttcacagag cttctaaagc aaacaagaag ccagttcagt tacctcgggg aatggtgaag

901 tcactattat atcagatcct agatggtatt cactacctgc atgctaactg ggtgttgcac

961 agagatttga aacctgctaa tattttagtt atgggtgaag gtcctgagcg aggaagagta

1021 aaaattgctg acatgggctt tgcccgatta tttaattcac ctttgaagcc tttagcagat

1081 ttggatccag tggttgttac attctggtac cgagcccctg aactacttct tggagcaagg

1141 cattatacca aagctattga tatttgggct atagggtgta tatttgcaga actactaacg

1201 tcagaaccaa tatttcactg tcgacaagag gacatcaaaa ctagtaatcc ttatcaccat
 
1261 gaccagctgg acagaatatt caatgtaatg ggatttcctg cagataaaga ttgggaagat

1321 ataaaaaaga tgcctgaaca ttcaacatta atgaaagatt tcagaagaaa tacgtatacc

1381 aactgcagcc ttatcaagta tatggaaaaa cataaagtta aaccagatag taaagcattc

1441 cacttgcttc agaagctgct taccatggac ccaataaagc gaattacctc agaacaggct

1501 atgcaggacc cctatttctt agaagaccca cttcctacat cagacgtttt tgccggttgt

1561 caaatccctt acccaaaacg agaattttta acggaagaag aacctgatga caaaggagac

1621 aaaaagaacc agcagcagca gcagggcaat aaccacacta atggaactgg ccacccaggg

1681 aatcaagaca gcagtcacac acagggaccc ccgttgaaga aagtgagagt tgttcctcct

1741 accactacct caggtggact tatcatgacc tcagactatc agcgttccaa tccacatgct

1801 gcctatccca accctggacc aagcacatca cagccgcaga gcagcatggg atactcagct

1861 acctcccagc agcctccaca gtactcacat cagacacatc ggtactgagc tgcatcggaa

1921 tcttgtccat gcactgttgc gaatgctgca gggctgactg tgcagctctc tgcgggaacc

1981 tggtatgggc catgagaatg tactgtacaa ccacatcttc aaaatgtcca gtagccaagt

2041 tccaccactt ttcacagatt ggggtagtgg cttccaagtt gtacctattt tggagttaga

2101 cttgaaaaga aagtgctagc acagtttgtg ttgtggattt gctacttcca tagtttactt

2161 gacatggttc agactgacca atgcattttt ttcagtgaca gtctgtagca gttgaagctg

2221 tgaatgtgct aggggcaagc atttgtcttt gtatgtggtg aattttttca gtgtaacaac

2281 attatctgac caatagtaca cacacagaca caaagtttaa ctggtacttg aaacatacag

2341 tatatgttaa cgaaataacc aagactcgaa atgagattat tttggtacac ctttcttttt

2401 agtgtcttat cagtgggctg attcattttc tacattaatc agtgttttct gaccaagaat

2461 attgcttgga tttttttgaa agtacaaaaa gccacatagt ttttccagaa aggtttcaaa

2521 actcccaaag attaacttcc aacttataag tttgttttta ttttcaatct atgacttgac

2581 tggtattaaa gctgctattt gatagtaatt aaatatgttg tcattgatat aaacctgttt

2641 ggttcagcaa acaaactaaa atgattgtca tagacagtgt tttatttttc ctgttggtgt
 
2701 tgctgatttg tgagcatgct ttaagatgaa aaaagcatga atgataactt ccttaaaaag

2761 gtgcggcatc caattcaaat attttcgtcc tgattttaaa gctggttggt gtagtgctat

2821 taaaatttcg ttcagttaat tttccttttg aaaacttgtt cgcacgttgt ttagggtgcc

2881 cttacttcag caaaggagaa ggagtaggag agccttagaa tttttgagga aaaaaaaacc

2941 tataacatac aatgtactgt atcaaactat tttacatgaa tgacacaagt attctgaata

3001 aaaaataatt gaacattgtt aaaaacaagg tgttatgtaa taaatttatt tttcataaat

3061 caaaa
**Homo sapiens cyclin C (CCNC), transcript variant 1, mRNA (SEQ ID NO: 3) NCBI Reference Sequence: NM_005190.4:**

1 gtcgccgccg cggagcggag tcgagccgag ctgatttgat cgaggagcgc ggttaccgga

61 cgggctgggt ctatggtcgc tccgcgggcc gctccgccgg ctggtgcttt tttatcaggg

121 caagctgtgt tccatggcag ggaacttttg gcagagctcc cactatttgc aatggatttt

181 ggataaacaa gatctgttga aggagcgcca aaaggattta aagtttctct cagaggaaga

241 atattggaag ttacaaatat tttttacaaa tgttatccaa gcattaggtg aacatcttaa

301 attaagacaa caagttattg ccactgctac ggtatatttc aagagattct atgccaggta

361 ttctctgaaa agtatagatc ctgtattaat ggctcctaca tgtgtgtttt tggcatccaa

421 agtagaggaa tttggagtag tttcaaatac aagattgatt gctgctgcta cttctgtatt

481 aaaaactaga ttttcatatg cctttccaaa ggaatttcct tataggatga atcatatatt

541 agaatgtgaa ttctatctgt tagaactaat ggattgttgc ttgatagtgt atcatcctta

601 tagacctttg ctccagtatg tgcaggacat gggccaagaa gacatgttgc ttccccttgc

661 atggaggata gtgaatgata cctacagaac ggatctttgc ctactgtatc ctcctttcat

721 gatagcttta gcttgcctac atgtagcctg tgttgtacag cagaaagatg ccaggcaatg

781 gtttgctgag ctttctgtgg atatggaaaa gattttggaa ataatcaggg ttattttaaa

841 actatatgag cagtggaaga atttcgatga gagaaaagag atggcaacca ttcttagtaa

901 gatgccaaaa ccaaaaccac ctccaaacag tgaaggagag cagggtccaa atggaagtca

961 gaactctagc tacagccaat cttaaaacat tccgaagaat tccatagtgg accacttgga

1021 aataaaccat tggacagatt tcagtaatgt cttcagtgga acacaaatga aaatgaatag

1081 cttgtttctg tcaagcatat tggaaagtga ttttattttt gcaaaataag tttttcttta

1141 atatgattct agtacataat tgattaaaat ctcttgatta taaatgtttg gaaaggttct

1201 aaggggacct acagacagac atacatagac atttcaaaat taatagcttt tgattagtat

1261 aatatttctt aatttggata ataaaaattg tagcttttta ttaagccagg aaacatgaag

1321 cataatttgt ttaaaattct ctttggtcat tgagggacca aaaaaggacg taaaatttac

1381 agtcaatcta tgagggtttt tttccctcca taagtttaac tttaaaactg tatttaagga

1441 atcaaatctt acaaaatcct ggaagatttt ggtaatgatg ttgataattt cagggaaatt

1501 aatcaagtac ctatattgat ttaaaagtgt attttattca gtagtttgag gatcttgcca

1561 tggaagatac tagcccagcc tagcagaaaa gtgcaatatg tatagcatac tttgacattt

1621 taaacatgat agtccataac cattttgaaa tgctgggcaa actacatgaa gttatttata

1681 attaattcac agctaatcag gcattttgaa agcttaattg gattcaaaaa ccataatgtt

1741 ggaatttggt aaaattttaa tgttgatttt tactgtgaaa aggtttttat aagatataca

1801 caccctagtt taatgttgtg tcttggtgtg gatttacaga tttactacag gtattctgaa

1861 ccaggaacac aatcaggttt caggccagtt tgatactggc tgtccttaat tctaatatga

1921 gagtaggaca tcatactaaa tgttatgtca gtgggactgt actgtctgtg gaacttagca

1981 aattaatcat tttcttcaga cttgaaggag agtgataaat aaaatttgga gtcataggat

2041 attgatgcac aatttaagga ttaaacattt ttaatcaatt gtggatgatg gcttattaaa

2101 tgttgacttc ctagtataaa actgcaagaa taaaagtaac ttctccagct gta

### CDC7 siRNA:

Example target sequences for siRNA oligos specific for Cdc7 coding region include, but is not an exhaustive list: 5'-aagctcagcaggaaaggtgtt-3' (SEQ ID NO: 4) (Cdc7-A); 5'-aaagaagttccagcacaagac-3' (SEQ ID NO: 5) (Cdc7-B); and 5'-aagcagtcaaagactgtggat-3' (SEQ ID NO: 6) (Cdc7-D) (Montagnoli et al., 2004).

### CDK8 and CCNC siRNA:

Example target sequences for siRNA oligos specific for CDK8 and CCNC coding region include, but is not an exhaustive list: 5'-GCAACCAUUCUUAGUAAGAUGCCAA-3' (SEQ ID NO: 7) (CCNC #1), 5'-GGAAUUUCCUUAUAGGAUGAAUCAT-3' (SEQ ID NO: 8) (CCNC #2), 5'-AGGCAUUAUACCAAACGUAUUGATA-3' (SEQ ID NO: 9) (CDK8 #1), 5'-GUAAAGCAUUCCACUUGCUUCAGAA-3' (SEQ ID NO: 10) (CDK8 #2) (Lloyd et al., 2021).

### CCNC shRNA:

Example target sequences for lentiviral shRNA constructs generated by the RNAi consortium (GPP Web Portal - Home (broadinstitute.org)). Some of these shRNA sequences have been used to target CDK8 and CCNC (Firestein et al., 2008) and are also commercially available through sigma-aldrich/Merck. This is not an exhaustive list.

| Gene Symbol | Taxon | Gene ID | Clone ID | Target Seq |
|---|---|---|---|---|
| CDC7 | human | 8317 | TRCN0000350364 | TTCAAGAAGTACGGGAATATA (SEQ ID NO: 11) |
| CDC7 | human | 8317 | TRCN0000003172 | CGCATTCATCAGTTTGGTATT (SEQ ID NO: 12 |
| CDC7 | human | 8317 | TRCN0000320935 | CGCATTCATCAGTTTGGTATT (SEQ ID NO: 13) |
| CDC7 | human | 8317 | TRCN0000196970 | GCTGACAGATTTCCCATTTAG (SEQ ID NO: 14) |
| CDC7 | human | 8317 | TRCN0000320938 | AGAGACCAGAGCAGGATTAAT (SEQ ID NO: 15) |
| CDC7 | human | 8317 | TRCN0000382344 | GCTGTACCACAGCTTAGTAAT (SEQ ID NO: 16) |
| CDC7 | human | 8317 | TRCN0000195201 | CCAATCAAACTACAGCAATTG (SEQ ID NO: 17) |
| CDC7 | human | 8317 | TRCN0000196542 | GAAGCTTTGTTGCATCCATTT (SEQ ID NO: 18) |
| CDC7 | human | 8317 | TRCN0000320936 | GAAGCTTTGTTGCATCCATTT (SEQ ID NO: 19) |
| CDC7 | human | 8317 | TRCN0000003171 | CCTAATCTGTTTGGTAAGTAT SEQ ID NO: 20) |
| CDC7 | human | 8317 | TRCN0000003169 | CGTGATGTTAAGCCCAGCAAT (SEQ ID NO: 21) |
| CDC7 | human | 8317 | TRCN0000003168 | GCCACAGCACAGTTACAAGTA (SEQ ID NO: 22) |
| CDC7 | human | 8317 | TRCN0000320863 | GCCACAGCACAGTTACAAGTA (SEQ ID NO: 23) |
| CDC7 | human | 8317 | TRCN0000003170 | CCAGGACAATACTCAGGGAAT (SEQ ID NO: 24) |
| CDC7 | human | 8317 | TRCN0000196543 | GAATACAGAATTGACGGTATT (SEQ ID NO: 25) |
| CDK8 | human | 1024 | TRCN0000382350 | ATGGTGAAGTCACTATTATAT (SEQ ID NO: 26) |
| CDK8 | human | 1024 | TRCN0000322391 | CATGGGCTTTGCCCGATTATT (SEQ ID NO: 27) |
| CDK8 | human | 1024 | TRCN0000322410 | CTAACGTCAGAACCAATATTT (SEQ ID NO: 28) |
| CDK8 | human | 1024 | TRCN0000350308 | CTAACGTCAGAACCAATATTT (SEQ ID NO: 29) |
| CDK8 | human | 1024 | TRCN0000196569 | GAATGGTGAAGTCACTATTAT (SEQ ID NO: 30) |
| CDK8 | human | 1024 | TRCN0000350344 | GCTTACCATGGACCCAATAAA (SEQ ID NO: 31) |
| CDK8 | human | 1024 | TRCN0000195594 | CACTACCTCAGGTGGACTTAT (SEQ ID NO: 32) |
| CDK8 | human | 1024 | TRCN0000322329 | TTTGGGCTATAGGGTGTATAT (SEQ ID NO: 33) |
| CDK8 | human | 1024 | TRCN0000199980 | GTCCATGCACTGTTGCGAATG (SEQ ID NO: 34) |
| CDK8 | human | 1024 | TRCN0000196702 | GCTATTGATATTTGGGCTATA (SEQ ID NO: 35) |
| CDK8 | human | 1024 | TRCN0000000493 | CACTTCCTACATCAGACGTTT (SEQ ID NO: 36) |
| CDK8 | human | 1024 | TRCN0000362345 | TATATTTGCAGAACTACTAAC (SEQ ID NO: 37) |
| CDK8 | human | 1024 | TRCN0000195463 | CGAGAGCTTAAGCATCCAAAC (SEQ ID NO: 38) |
| CDK8 | human | 1024 | TRCN0000000490 | GCAGGGCAATAACCACACTAA (SEQ ID NO: 39) |
| CDK8 | human | 1024 | TRCN0000320651 | GCAGGGCAATAACCACACTAA (SEQ ID NO: 40) |
| CDK8 | human | 1024 | TRCN0000194708 | CATGAGAATGTACTGTACAAC (SEQ ID NO: 41) |
| CDK8 | human | 1024 | TRCN0000000491 | CCTCTGGCATATAATCAAGTT (SEQ ID NO: 42) |
| CDK8 | human | 1024 | TRCN0000023264 | GCCTTATCAAGTATATGGAAA (SEQ ID NO: 43) |
| CDK8 | human | 1024 | TRCN0000023108 | GCAGATAAAGATTGGGAAGAT (SEQ ID NO: 44) |
| CDK8 | human | 1024 | TRCN0000023106 | CCCGATTATTTAATTCACCTT (SEQ ID NO: 45) |
| CDK8 | human | 1024 | TRCN0000000492 | CAAGGCATTATACCAAAGCTA (SEQ ID NO: 46) |
| CDK8 | human | 1024 | TRCN0000000489 | ATGTCCAGTAGCCAAGTTCCA (SEQ ID NO: 47) |
| CDK8 | human | 1024 | TRCN0000320652 | ATGTCCAGTAGCCAAGTTCCA (SEQ ID NO: 48) |
| CDK8 | human | 1024 | TRCN0000199779 | GAACCTGGTATGGGCCATGAG (SEQ ID NO: 49) |
| CCNC | human | 892 | TRCN0000304592 | TCTAGCTACAGCCAATCTTAA (SEQ ID NO: 50) |
| CCNC | human | 892 | TRCN0000077831 | GCATTAGGTGAACATCTTAAA (SEQ ID NO: 51) |
| CCNC | human | 892 | TRCN0000020189 | GCATCCAAAGTAGAGGAATTT (SEQ ID NO: 52) |
| CCNC | human | 892 | TRCN0000319164 | GCATCCAAAGTAGAGGAATTT (SEQ ID NO: 53) |
| CCNC | human | 892 | TRCN0000020193 | CCCACTATTTGCAATGGATTT (SEQ ID NO: 54) |
| CCNC | human | 892 | TRCN0000077830 | CATGGAGGATAGTGAATGATA (SEQ ID NO: 55) |
| CCNC | human | 892 | TRCN0000302106 | CATGGAGGATAGTGAATGATA (SEQ ID NO: 56) |
| CCNC | human | 892 | TRCN0000020191 | GCATGGAGGATAGTGAATGAT (SEQ ID NO: 57) |
| CCNC | human | 892 | TRCN0000319165 | GCATGGAGGATAGTGAATGAT (SEQ ID NO: 58) |
| CCNC | human | 892 | TRCN0000020190 | CCATTCTTAGTAAGATGCCAA (SEQ ID NO: 59) |
| CCNC | human | 892 | TRCN0000319166 | CCATTCTTAGTAAGATGCCAA (SEQ ID NO: 60) |
| CCNC | human | 892 | TRCN0000020192 | CTACGGTATATTTCAAGAGAT (SEQ ID NO: 61) |
| CCNC | human | 892 | TRCN0000319188 | CTACGGTATATTTCAAGAGAT (SEQ ID NO: 62) |

## Claims

1. A Cell Division Cycle 7 kinase inhibitor (CDC7i), wherein the CDC7i is administered in combination with:
i) a Cyclin Dependent Kinase 8 inhibitor (CDK8i), and/or
ii) a Cyclin C inhibitor (CCNCi);
for use in the treatment or prevention of cancer in a subject.

2. The CDC7i for the use of claim 1, wherein the CDC7i comprises or consists of any one of the group of CDC7 inhibitors selected from PHA-767491, XL413, AS-0141, Dequalinium Chloride, LY3143921, TQB3824, NMS-1116354, and TAK-931; and/or combinations thereof; or the CDC7i is an inhibitory RNA molecule capable of inhibiting the expression of CDC7.

3. The CDC7i for the use of claim 1 or claim 2, wherein the CDK8i comprises or consists of any one of the group of CDK8 inhibitors selected from BI1347, RVU120, Senexin A, Senexin B, Senexin C, MSC2530818, AS2863619, 16-didehydro-cortistatin A, BRD6989, CCT-251545, CCT-251921, TSN084, JH-XVI-178, and JH-XI-10-02; and/or combinations thereof; or the CDK8i is an inhibitory RNA molecule capable of inhibiting the expression of CDK8.

4. The CDC7i for the use according to any preceding claim, wherein the CCNCi is an inhibitory RNA molecule capable of inhibiting the expression of CCNC.

5. A composition comprising a CDC7 inhibitor and a CDK8 and/or CCNC inhibitor.

6. A kit, wherein the kit comprises:
i) a CDC7 inhibitor; and
ii) a CDK8 and/or CCNC inhibitor.

7. A composition according to claim 5 or kit according to claim 6 for use as a medicament.

8. The composition or kit for the use according to claim 7, wherein the use is for treatment or prevention of cancer in a subject.

9. A Cell Division Cycle 7 kinase inhibitor (CDC7i) for use in a method of treatment or prevention of cancer in a subject, wherein the cancer is determined or known to be deficient in functional CDK8 and/or CNCC expression.

10. The CDC7i for the use according to claim 9, wherein the cancer is selected from Diffuse Large B-Cell Lymphoma; Sarcoma; Lung Adenocarcinoma; Prostate Adenocarcinoma; Uveal Melanoma; Liver Hepatocellular Carcinoma; Stomach Adenocarcinoma; and Bladder Urothelial Carcinoma.

11. Use of CDK8 and/or CCNC as a biomarker for predicting the efficacy of CDC7i therapy for cancer of a subject, wherein a reduction or absence of expression of functional CDK8 and/or CCNC in a subject with respect to the average level in a population of subjects having the same cancer type and/or stage is indicative of an increased efficacy of the CDC7i therapy for cancer.

12. A method of predicting the efficacy of a CDC7i therapy for cancer in a subject, the method comprising determining the levels of expression of functional CDK8 and/or CCNC in the subject,
wherein a decreased level of expression of CDK8 and/or CCNC in the subject with respect to the average level in a population of subjects having the same cancer type and/or stage is indicative of a more effective CDC7i therapy for the cancer in the subject.

13. A method of selecting a subject for treatment for cancer with a CDC7i, the method comprising determining the levels of expression of functional CDK8 and/or CCNC in the subject,
wherein the subject is selected for CDC7i treatment if they have a decreased level of expression of CDK8 and/or CCNC with respect to the average level in a population of subjects having the same cancer type and/or stage.

## Patentansprüche

1. Zellteilungszyklus-7-Kinaseinhibitor (CDC7i), wobei der CDC7i in Kombination mit Folgendem verabreicht wird:
i) einem Cyclin-abhängigen Kinase-8-Inhibitor (CDK8i) und/oder
ii) einen Cyclin-C-Inhibitor (CCNCi);
zur Verwendung bei der Behandlung oder Vorbeugung von Krebs bei einem Patienten.

2. CDC7i für die Verwendung nach Anspruch 1, wobei das CDC7i einen beliebigen CDC7-Inhibitor aus der Gruppe der CDC7-Inhibitoren, ausgewählt aus PHA-767491, XL413, AS-0141, Dequaliniumchlorid, LY3143921, TQB3824, NMS-1116354 und TAK-931, und/oder Kombinationen davon umfasst oder daraus besteht; oder das CDC7i ein inhibitorisches RNA-Molekül ist, das die Expression von CDC7 hemmen kann.

3. CDC7i für die Verwendung nach Anspruch 1 oder 2, wobei das CDK8i einen beliebigen CDK8-Inhibitor aus der Gruppe der CDK8-Inhibitoren, ausgewählt aus BI1347, RVU120, Senexin A, Senexin B, Senexin C, MSC2530818, AS2863619, 16-Didehydro-Cortistatin A, BRD6989, CCT-251545, CCT-251921, TSN084, JH-XVI-178 und JH-XI-10-02, umfasst oder daraus besteht; und/oder Kombinationen davon; oder das CDK8i ist ein hemmendes RNA-Molekül, das die Expression von CDK8 hemmen kann.

4. CDC7i für die Verwendung nach einem der vorstehenden Ansprüche, wobei das CCNCi ein inhibitorisches RNA-Molekül ist, das die Expression von CCNC hemmen kann.

5. Zusammensetzung, umfassend einen CDC7-Inhibitor und einen CDK8- und/oder CCNC-Inhibitor.

6. Kit, wobei das Kit Folgendes umfasst:
i) einen CDC7-Inhibitor; und
ii) einen CDK8- und/oder CCNC-Inhibitor.

7. Zusammensetzung nach Anspruch 5 oder Kit nach Anspruch 6 zur Verwendung als Medikament.

8. Zusammensetzung oder Kit für die Verwendung nach Anspruch 7, wobei die Verwendung zur Behandlung oder Vorbeugung von Krebs in einem Subjekt dient.

9. Zellteilungszyklus-7-Kinaseinhibitor (CDC7i) zur Verwendung in einem Verfahren zur Behandlung oder Vorbeugung von Krebs bei einem Patienten, wobei bestimmt oder bekannt ist, dass der Krebs einen Mangel an funktioneller CDK8- und/oder CNCC-Expression aufweist.

10. CDC7i für die Verwendung nach Anspruch 9, wobei der Krebs ausgewählt ist aus diffusem großzelligem B-Zell-Lymphom, Sarkom, Lungenadenokarzinom, Prostataadenokarzinom, Aderhautmelanom, hepatozellulärem Leberkarzinom, Magenadenokarzinom und urothelialem Blasenkarzinom.

11. Verwendung von CDK8 und/oder CCNC als Biomarker zum Vorhersagen der Wirksamkeit der CDC7i-Therapie für Krebs eines Subjekts, wobei eine Verringerung oder das Fehlen der Expression von funktionellem CDK8 und/oder CCNC in einem Subjekt in Bezug auf das durchschnittliche Niveau in einer Population von Subjekten, die denselben Krebstyp und/oder dasselbe Krebsstadium haben, auf eine erhöhte Wirksamkeit der CDC7i-Therapie für Krebs hinweist.

12. Verfahren zum Vorhersagen der Wirksamkeit einer CDC7i-Therapie für Krebs bei einem Patienten, wobei das Verfahren das Bestimmen der Expressionsniveaus von funktionellem CDK8 und/oder CCNC bei dem Patienten umfasst,
wobei ein vermindertes Expressionsniveau von CDK8 und/oder CCNC in dem Subjekt in Bezug auf das durchschnittliche Niveau in einer Population von Subjekten mit demselben Krebstyp und/oder -stadium auf eine wirksamere CDC7i-Therapie für den Krebs in dem Subjekt hinweist.

13. Verfahren zum Auswählen eines Subjekts für die Behandlung von Krebs mit einem CDC7i, wobei das Verfahren das Bestimmen der Expressionsniveaus von funktionellem CDK8 und/oder CCNC in dem Subjekt umfasst,
wobei das Subjekt für die CDC7i-Behandlung ausgewählt wird, wenn es ein vermindertes Expressionsniveau von CDK8 und/oder CCNC in Bezug auf das durchschnittliche Niveau in einer Population von Subjekten mit dem gleichen Krebstyp und/oder Stadium aufweist.

## Revendications

1. Inhibiteur de la kinase du cycle de division cellulaire 7 (CDC7i), dans lequel le CDC7i est administré en association avec :
i) un inhibiteur de la kinase 8 dépendante des cyclines (CDK8i), et/ou
ii) un inhibiteur de la cycline C (CCNCi) ;
destiné à être utilisé dans le traitement ou la prévention du cancer chez un sujet.

2. CDC7i destiné à l'utilisation selon la revendication 1, dans lequel le CDC7i comprend ou est constitué de l'un quelconque du groupe d'inhibiteurs de CDC7 choisis parmi PHA-767491, XL413, AS-0141, le chlorure de déqualinium, LY3143921, TQB3824, NMS-1116354 et TAK-931 ; et/ou des combinaisons de ceux-ci ; ou le CDC7i est une molécule d'ARN inhibiteur capable d'inhiber l'expression de CDC7.

3. CDC7i destiné à l'utilisation selon la revendication 1 ou la revendication 2, dans lequel le CDK8i comprend ou est constitué de l'un quelconque du groupe d'inhibiteurs de CDK8 choisis parmi BI1347, RVU120, Sénexine A, Sénexine B, Sénexine C, MSC2530818, AS2863619, 16-didéhydro-cortistatine A, BRD6989, CCT-251545, CCT-251921, TSN084, JH-XVI-178 et JH-XI-10-02 ; et/ou des combinaisons de ceux-ci ; ou le CDK8i est une molécule d'ARN inhibiteur capable d'inhiber l'expression de CDK8.

4. CDC7i destiné à l'utilisation selon l'une quelconque revendication précédente, dans lequel le CCNCi est une molécule d'ARN inhibiteur capable d'inhiber l'expression de CCNC.

5. Composition comprenant un inhibiteur de CDC7 et un inhibiteur de CDK8 et/ou de CCNC.

6. Kit, dans lequel le kit comprend :
i) un inhibiteur de CDC7 ; et
ii) un inhibiteur de CDK8 et/ou de CCNC.

7. Composition selon la revendication 5 ou kit selon la revendication 6 destiné(e) à être utilisé(e) comme médicament.

8. Composition ou kit destiné(e) à l'utilisation selon la revendication 7, dans laquelle/lequel l'utilisation est destinée au traitement ou à la prévention du cancer chez un sujet.

9. Inhibiteur de la kinase du cycle de division cellulaire 7 (CDC7i) destiné à être utilisé dans un procédé de traitement ou de prévention du cancer chez un sujet, dans lequel il est établi ou connu que le cancer présente un déficit en expression de CDK8 et/ou de CNCC fonctionnels.

10. CDC7i destiné à l'utilisation selon la revendication 9, dans lequel le cancer est sélectionné parmi le lymphome diffus à grandes cellules B ; le sarcome ; l'adénocarcinome pulmonaire ; l'adénocarcinome de la prostate ; le mélanome uvéal ; le carcinome hépatocellulaire ; l'adénocarcinome de l'estomac ; et le carcinome urothélial de la vessie.

11. Utilisation de CDK8 et/ou de CCNC comme biomarqueur permettant de prédire l'efficacité d'un traitement par CDC7i contre le cancer d'un sujet, dans laquelle une réduction ou une absence d'expression de CDK8 et/ou de CCNC fonctionnels chez un sujet par rapport au niveau moyen dans une population de sujets ayant le même type et/ou stade de cancer indique une efficacité accrue du traitement par CDC7i contre le cancer.

12. Procédé permettant de prédire l'efficacité d'un traitement par CDC7i contre le cancer chez un sujet, le procédé comprenant la détermination des niveaux d'expression de CDK8 et/ou de CCNC fonctionnels chez le sujet,
dans lequel un niveau d'expression réduit de CDK8 et/ou de CCNC chez le sujet par rapport au niveau moyen dans une population de sujets ayant le même type et/ou stade de cancer indique un traitement par CDC7i plus efficace contre le cancer chez le sujet.

13. Procédé de sélection d'un sujet pour un traitement contre le cancer avec un CDC7i, le procédé comprenant la détermination des niveaux d'expression de CDK8 et/ou de CCNC fonctionnels chez le sujet,
dans lequel le sujet est sélectionné pour un traitement par CDC7i s'il a un niveau d'expression réduit de CDK8 et/ou de CCNC par rapport au niveau moyen dans une population de sujets ayant le même type et/ou stade de cancer.
